# EUROPEAN PATENT APPLICATION

(11) **EP 3 763 390 A1**
(43) Date of publication of application: **13.01.2021**
(21) Application number: 20185581.4
(22) Date of filing: 13.07.2020
(51) Int. Cl.: A61K 47/54, A61P 35/00

(54) **ARTEMISININ DERIVATIVES**

(30) Priority: 12.07.2019 US 201962873151 P; 12.07.2019 US 201962873293 P
(71) Applicant: Da Zen Theranostics Inc., San Jose, California 95123 (US)
(72) Inventor: CHUNG, Leland W.K., Beverly Hills, California 90210 (US); CHU, Gina C.Y., San Diego, California 92130 (US); ZHANG, Yi, Los Angeles, California 90063 (US)
(74) Representative: Ellis, Michael James

(57) **Abstract**

The present invention generally relates to artemisinin/dihydroartemisinin (DHA) derivatives, and their use for therapy, in particular cancer therapy. These tumor-homing artemisinin derivatives (THAD) comprise three moieties: an artemisinin/DHA or a derivative thereof, a heptamethine carbocyanine dye (HMCD) residue, and a linker that conjugates the HMCD dye residue to the artemisinin residue. The THAD include compounds wherein the linker is linked to one or two DHA residue(s) via one or more ether bonds, and wherein the linker is linked to two DHA residues via two bonds independently selected from ester, carbamate and thiocarbamate. The THAD of the invention provide improved growth inhibition of cancer cells. The present invention also relates to improved methods of cancer therapy wherein a THAD is administered to a cancer patient. In embodiments, one or more THAD may be co-administered in a coordinated administration schedule. Advantages of the THAD and their use include, among others, improved dose-response and/or efficacy. The invention also relates to new dyes, their drug conjugates, and processes of making them.

## Description

### FIELD OF THE INVENTION

The present invention generally relates to artemisinin/dihydroartemisinin (DHA) derivatives, and their use for therapy, in particular cancer therapy. These tumor-homing artemisinin derivatives (THAD) comprise three moieties: an artemisinin/DHA or a derivative thereof, a heptamethine carbocyanine dye (HMCD) residue, and a linker that conjugates the HMCD dye residue to the artemisinin residue. The THAD include compounds wherein the linker is linked to one or two DHA residue(s) via one or more ether bonds, and wherein the linker is linked to two DHA residues via two bonds independently selected from ester, carbamate and thiocarbamate. The THAD of the invention provide improved growth inhibition of cancer cells. The present invention also relates to improved methods of cancer therapy wherein a THAD is administered to a cancer patient. In embodiments, one or more THAD may be co-administered in a coordinated administration schedule. Advantages of the THAD and their use include, among others, improved dose-response and/or efficacy. The invention also relates to new dyes, their drug conjugates, and processes of making them.

### BACKGROUND OF THE INVENTION

Many cancer drugs are known and include various standard treatments such as tyrosine kinase inhibitors (TKI) and classic chemotherapeutics. Many such treatments are associated with significant side effects and/or subject to the development of drug resistance. Drugs effective for the treatment of advanced or metastatic cancers (or cancers that typically develop metastases) are few and treatment options for these cancers are very limited. Furthermore these cancer types often develop resistance in response to treatment with a given cancer drug.

Artemisinin and its derivatives, e.g. artesunate and artemether, and their active metabolites, in particular dihydroartemisinin, are a group of drugs typically used against malaria and parasitic worm infections. These sesquiterpene lactones contain an unusual peroxide bridge in their endoperoxide 1,2,4-trioxane ring, which is responsible for the drug's mechanism of action. They suffer from low bioavailability, poor pharmacokinetic properties, and development of resistance, and in therapy are typically used in combination with other drugs. Artemisinin and its derivatives have also been researched for potential anti-cancer effects in various cancers, particularly in combination with various chemotherapeutics, to enhance the effects thereof.

Heptamethine carbocyanine dyes (HMCD) are known for imaging e.g. of the human body for various diagnostic purposes; some of these dyes have been described for use in both cancer imaging as well as cancer therapy.

WO 2018/075996, WO 2018/075994 and WO 2018/075993 disclose certain drug-conjugated HMCD dyes ("DZ1") conjugated to certain resistance-prone drugs, and among others, Artemisinin, for drug-delivery to cancer cells, and the ability of these drug-releasing conjugates to re-sensitize drug-resistant cancer cells, and their co-administration with various resistance-prone therapeutics, including, among others, tyrosine kinases (e.g. Gefitinib or Icotinib), Cisplatin, Gemcitabine, Paclitaxel, Docetaxel, and the anti-androgens Enzalutamide and Abiraterone. The dye-drug conjugates include, among others, a dye conjugated via alkylester or alkylamide to Artemisinin; the ester conjugate is referred to herein-below as DZ1-DH-ester or mono-ester.

WO 2018/075994 discloses certain DZ1-drug conjugates linked via ester or amide bonds to Cisplatin, Simvastatin, or Artemisinin, and their use for sensitization to various resistance-prone therapeutics, including, among others, Cisplatin, Gemcitabine, Paclitaxel, and Docetaxel.

WO 2018/075993 discloses DZ1-drug conjugates linked via ester or amide bonds and their use in combination with administration of Tyrosine Kinase Inhibitors (TKI), such as Gefitinib or Icotinib, to sensitize cancer cells to TKI treatment and overcome TKI resistance.

Cancers treated with TKI include those particularly aggressive cancers that are prone to metastasize and/or develop drug resistance. However, a problem with TKI is the quick development of resistance to the TKI upon treatment. Another problem with some TKI is that despite the comparatively small size (MW of 300-600) the TKI may not, or not effectively, treat the brain. Other TKI that may treat the brain may have side effects or may be effective only in particular groups of patient.

Certain cancers are particularly difficult to treat, e.g. due to their aggressive growth, tendency to form metastases and/or develop resistance. For example, cancers of the kidney, e.g. renal cell carcinoma, are typically removed by surgery if possible, and neither chemotherapy nor other targeted therapies (such as TKI) have been shown to be very effective. Similarly, with regard cancers such as lung cancer or kidney cancer traditionally treated with TKI, improved treatments and therapeutics are needed to avoid development of resistance, and for patients previously treated with TKI, a treatment is needed that overcomes or is not affected by the developed TKI resistance, in particular for patients of e.g. clear cell renal cell carcinoma and non-small cell lung cancer (NSCLC), and including especially adenocarcinomas (AC) of the lung. With regard to cancers including lung cancers such as small cell lung cancer (SCLC) traditionally treated with chemotherapeutics, improved treatments and therapeutics are needed to avoid development of resistance, and for patients previously treated with a chemotherapeutic, therapeutics and treatments are needed that overcome or are not affected by the acquired resistance.

There remains a need for improved cancer therapeutics and cancer treatments, including improved effectiveness. In particular there remains a need for cancer therapeutics that provide a more rapid growth inhibition/cell death of cancer cells, and avoid the development of drug resistance. Furthermore, there remains a need for effective therapeutics and treatments with less side effects. Still further there remains a need for therapeutics and treatments effective at a lower dose. Also there remains a need for effective therapeutics and treatments with a reduced future risk, such as risk for cancer, metastasis and chemotherapeutical induced disease. Still further there remains a need for effective therapeutics and treatments that provide a less frequent administration schedule. Yet further there remains a need for effective therapeutics and treatments with less side effects and a less frequent administration. Also there is a need for therapeutics with a more favorable dose response curve. Further there is a need for therapeutics and treatments suitable for highly aggressive cancers, including those reduce or avoid administration of drugs that cause side effects. Also there is a need for improved therapeutics and treatments that do not require co-administration of drugs with undesirable side effects. In particular there is a need for improved therapeutics and treatments that do not require co-administration of chemotherapeutics with undesirable side effects, including general cytotoxicity (including various non-cancer cells).

Further, there remains a need for therapies and treatments that are suitable for a wide range of cancers, in particular including drug-resistant cancers, metastatic cancers, fast- growing cancers, and otherwise aggressive cancers, and various cancers with limited treatment options, including e,g, kidney cancer, prostate cancer and lung cancer. Also there is a need for improved therapeutics and treatments that cross the barrier to solid tumors or tumors present in encapsulated organs, and allow to treat such less-accessible tumors, e.g. kidney tumors. Further, there is a need for improved therapeutics and treatments that cross the blood-brain-barrier (BBB) and allow to treat brain tumors and metastases in the brain. These and other features and advantages of the present invention will be explained and will become apparent to one skilled in the art through the summary of the invention that follows.

### SUMMARY OF THE INVENTION

Aspects of the present invention are set out in the appended independent claims. Variations of these aspects are set out in the dependent claims.

Further aspects of the invention are described herein along with features and advantages of the invention which will become apparent from the following description, which is made with reference to the accompanying drawings.

### BRIEF DESCRIPTION OF THE DRAWINGS

**FIG. 1** shows DZ1-DHA-ether and MHI-148-bis-DHA-bis-ester, and for comparative purposes DZ1-DHA, a mono-ester.
**FIG. 2A** shows improved dose response and growth inhibition of kidney cancer cells.
**FIG. 2B** shows improved dose response and growth inhibition of Enzalutamide-resistant prostate cancer cells.
**FIG. 3A** shows growth inhibition of different prostate cancer cells.
**FIG. 3B** shows growth inhibition of lung and pancreatic cancer cells.
**FIG. 4A** shows inhibition of tumors in vivo in a human prostate tumor model.
**FIG. 5A** shows DZ3a co-localizes with mitochondria and lysosomes.
**FIG. 5B** shows DZ3a induces DNA damage and depletes mitochondria.
**FIG. 5C** shows DZ3a lowers the mitochondrial oxygen consumption rate ("OCR").
**FIG. 5D** shows induction of depolarization of mitochondrial membrane potential.
**FIG. 5E** shows induction of apoptosis and its blocking by inhibitors.
**FIG. 5F** shows induction of lipid peroxidation and mitochondrial ROS.
**FIG. 5G** shows decreased cellular GSH levels.
**FIG. 6A** illustrates the synthesis of a DZ1-DHA-ether (DZ3c).
**FIG. 6B** illustrates the synthesis of a MHI148-bis-DHA-ester (DZ3b).
**FIG. 6C** illustrates the synthesis of a DZ1a-bis-DHA-ether (DZ3d).
**FIG. 6D** illustrates the synthesis of a DZ1b-DHA-carbamate (DZ3e).
**FIG. 6E** illustrates the synthesis of a DZ1c-bis-DHA-carbamate (DZ3f).
**FIG. 6F** illustrates the synthesis of a DZ1b-DHA-thiocarbamate (DZ3g).
**FIG. 6G** illustrates the synthesis of a DZ1c-bis-DHA-thiocarbamate (DZ3h).

### DETAILED DESCRIPTION OF THE INVENTION

The present invention generally relates to artemisinin derivatives, and their use for therapy, in particular cancer therapy. These tumor-homing artemisinin derivatives (THAD) comprise three moieties: an artemisinin or a derivative thereof, a heptamethine carbocyanine dye (HMCD) residue, and a linker that conjugates the HMCD dye residue to the artemisinin residue. Specifically, embodiments of the invention include THAD compounds wherein the linker is linked to one or two artemisinin residue(s) via one or more ether bonds, and wherein the linker is linked to two artemisinin residues via two bonds independently selected from ester, carbamate and thiocarbamate. The THA of the invention provide improved growth inhibition of cancer cells. The present invention also relates to improved methods of cancer therapy wherein a THA is administered to a cancer patient. Advantages of the THAD and their use include, among others, improved efficacy and dose-response. These and other advantages of the THAD may allow for an improved administration schedule with less frequent instances of drug administration. Further, one or more THAD and optionally an HMCD-DHA-mono-ester may be co-administered in a coordinated administration schedule, wherein for example a maintenance dose of a HMCD-DHA-mono-ester or the bis-ester THAD may be provided with a loading dose of a bis-ether, bis-carbamate or bis-thiocarbamate THAD.

In embodiments, provided are THAD selected from the group consisting of formulae FI, FII, FIII and FIV as shown below: 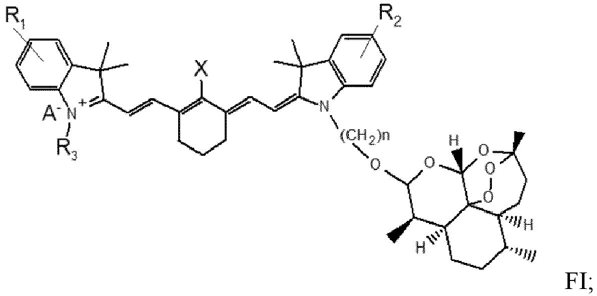 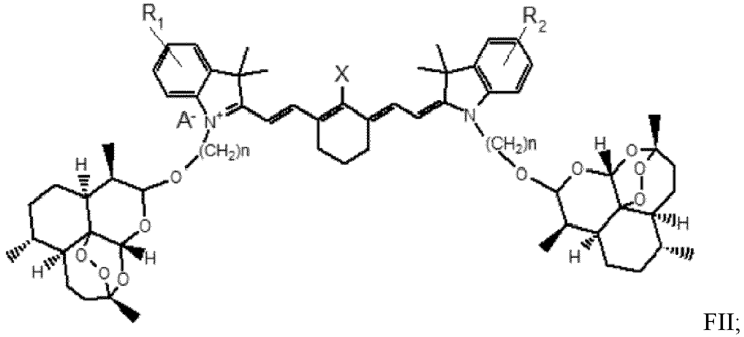 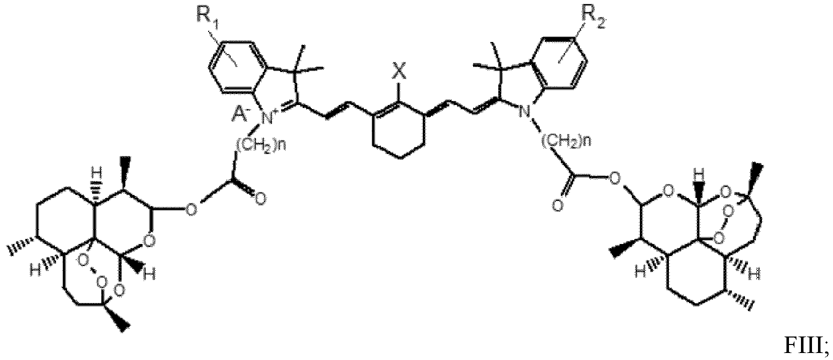 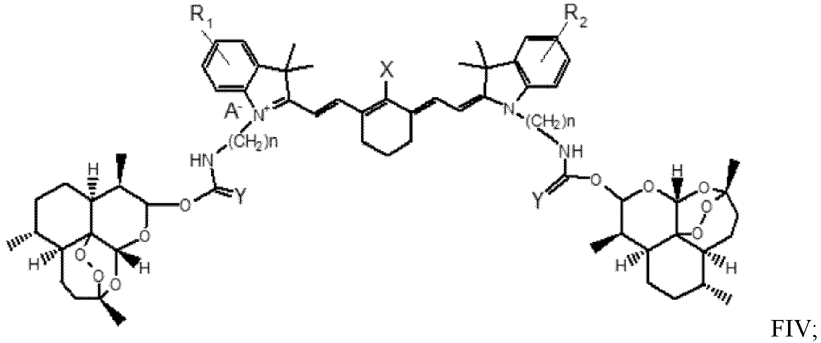 wherein X is a halogen residue; wherein n is independently selected from the group consisting of 2, 3, 4, 5, 6, 7, 8, 9, 10, 11, 12, 13, 14, 15, 16, 17 ,18, 19, 20; wherein the A⁻ group is a pharmaceutically acceptable negatively charged anion; wherein R₁ and R₂ are residues independently selected from the group consisting of: hydrogen, C₁-C₂₀ alkyl, sulphonate, C₁-C₂₀ alkylcarboxyl, C₁-C₂₀ alkylamino, C₁-C₂₀ aryl, -SO₃H, -PO₃H, -OH, - NH₂, and a halogen residue; wherein R₃ of formula FI is a residue selected from the group consisting of: C₁-C₂₅ alkyl, C₅-C₂₅ aryl, C₁-C₂₅ aralkyl, C₁-C₂₅ alkylsulphonate, C₁-C₂₅ alkylcarboxyl, C₁-C₂₅ alkylamino, C₁-C₂₅ co-alkylaminium, C₁-C₂₅ co-alkynyl, a PEGyl polyethylene chain with (-CH₂-CH₂-O-)₂₋₂₀, a PEGylcarboxylate with (-CH₂-CH₂-O-)₂₋₂₀, a ω-PEGylaminium with (-CH₂-CH₂-O-)₂₋₂₀, a ω-acyl-NH, a ω- acyl-lysinyl-, a ω -acyl-triazole, a ω- PEGylcarboxyl-NH- with (-CH₂-CH₂-O-)₂₋₂₀, a ω-PEGylcarboxyl-lysinyl with (-CH₂-CH₂-O-)₂₋₂₀, and a ω- PEGylcarboxyl-triazole with (-CH₂-CH₂-O-)₂₋₂₀; and wherein Y of formula FIV is independently selected from O and S.

In embodiments, provided are ether-linked THAD (mono-ether mono DHA, bis-ether bis-DHA) that are THAD selected from the group consisting of a THAD of formula FI and FII.

In embodiments, provided are bis-DHA THAD (bis-ether, bis-ester, bis-carbamate/thiocarbamate bis-DHA) that are THAD selected from the group consisting of a THAD of formulae FII, FIII and FIV.

In embodiments, provided are THAD wherein X is Cl; and/or wherein one or more of R₁ and R₂ is H; and/or wherein R₃ is a -(CH₂)n-SO₃⁻ alkylsulphonate residue, wherein n of R₁ is selected from 2, 3, 4, 5, 6, 7 and 8; and/or wherein R₃ is a -(CH₂)₄-SO₃⁻ alkylsulphonate residue.

In embodiments, provided are pharmaceutical compositions comprising one or more THAD and one or more pharmaceutical excipient, wherein the one or more THAD is selected from the group consisting of: a THAD of formula FI as described herein, a THAD of formula FII as defined herein, a THAD of formula FIII as described herein, a THAD of formula FIV as described herein. Without limitation, these THAD comprise, e.g., ether-linked THAD and/or bis-DHA THAD. Further, without limitation, these THAD comprise THAD wherein X is Cl; and/or wherein one or more of R₁ and R₂ is H; and/or wherein R₃ is a -(CH₂)n-SO₃⁻ alkylsulphonate residue, wherein n of R₁ is selected from 2, 3, 4, 5, 6, 7 and 8; and/or wherein R₃ is a -(CH₂)₄-SO₃⁻ alkylsulphonate residue.

In embodiments, provided are pharmaceutical compositions wherein the composition is provided in a dosage form which is adapted to provide a low dosage of up to 2 mg/kg of the one or more THAD or less upon administration of the dosage form, e.g. 1 mg/kg or less, 0.5 mg/kg or less, 0.25 mg/kg or less, or 0.1 mg/kg or less.

In embodiments, provided are methods of treating cancer wherein one or more THAD as described herein is administered to a patient in need thereof in amounts sufficient to inhibit cancer cell or pre-cancerous cell growth or induce apoptosis in cancer or pre-cancerous cells in the patient.

In embodiments, provided are methods wherein the one or more THAD as described herein is provided in a low dosage of up to 2 mg/kg of the one or more THAD or less to a patient, e.g. 1 mg/kg or less, 0.5 mg/kg or less, 0.25 mg/kg or less, or 0.1 mg/kg or less.

In embodiments, provided are methods wherein the one or more THAD as described herein is co-administered in a coordinated administration schedule together with one or more secondary drug, and wherein the one or more secondary drug is selected from the group consisting of: a hormonal antagonist, an anti-androgenic drug, Abiraterone, Enzalutamid, a chemotherapeutic drug, Docetaxel, Paclitaxel, and Cabazitaxel.

In embodiments, provided are methods wherein the one or more THAD is administered to a patient whose cancer cells, pre-cancerous lesions, tissues, tumors or metastases are identified to carry one or more genetic aberration in one or more gene encoding for one or more tyrosine kinase receptor, selected from the group comprising: epidermal growth factor receptor tyrosine kinase (EGFR), Anaplastic lymphoma kinase receptor (ALF), and Proto-oncogene tyrosine-protein kinase (ROS or ROS1).

In embodiments, provided are methods wherein the one or more THAD is administered to a patient whose cancer cells, pre-cancerous lesions, tumors or metastases have acquired resistance to one or more tyrosine kinase inhibitor (TKI), including a patient who received prior TKI treatment with one or more TKI prior to ELSD administration and whose response to the prior TKI treatment is therapeutically insufficient.

In embodiments, provided are methods wherein the TKI is selected from the group consisting of an epidermal growth factor receptor tyrosine kinase inhibitor (EGFR-TKI), an ALK tyrosine kinase receptor inhibitor (ALK-TKI), and an inhibitor to Proto-oncogene tyrosine-protein kinase ROS (ROS-TKI).

In embodiments, provided are methods wherein the EGFR-TKI is selected from the group consisting of: Gefitinib, Icotinib, Erlotinib, Brigatinib, Dacomitinib, Lapatinib, Vandetanib, Afatinib, Osimertinib (AZD9291), CO-1686, HM61713, Nazartinib (EGF816), Olmutinib, PF-06747775, YH5448, Avitinib (AC0010), Rociletinib, and Cetuximab.

In embodiments, provided are methods wherein the patient is suffering from a drug-resistant cancer as determined by drug exposure or genetic testing, the drug-resistant cancer selected from the group comprising: kidney cancer, prostate cancer, pancreatic cancer, lung cancer, non-small cell lung carcinoma (NSCLC; NSCLC may include squamous-cell carcinoma, adenocarcinoma (mucinous cystadenocarcinoma), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma), SCLC (small cell lung carcinoma), combined small-cell carcinoma, non-carcinoma cancers of the lung (sarcoma, lymphoma, immature teratoma, and melanoma), kidney cancer, lymphoma, colorectal cancer, skin cancer, HCC cancer, and breast cancer, squamous-cell carcinoma of the lung, anal cancers, glioblastoma, epithelial tumors of the head and neck, and other cancers.

In embodiments, provided are methods wherein the patient is a patient suffering from a drug-resistant lung cancer as determined by drug exposure or genetic testing, the drug resistant lung cancer selected from the group comprising: small cell carcinoma lung cancer (SCCLC), non-small cell lung carcinoma (NSCLC), combined small-cell carcinoma, squamous-cell carcinoma, adenocarcinoma (AC, mucinous cystadenocarcinoma, MCACL), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma, non-carcinoma cancer of the lung, sarcoma, lymphoma, immature teratoma, and melanoma.

In embodiments, provided are methods wherein the one or more THAD and one or more HMCD-DHA-mono ester, are co-administered in a coordinated administration schedule of one or more combined dosage forms, wherein each dosage form comprises: a) one or more HMCD-DHA-mono-ether and one or more HMCD-DHA-mono ester; b) one or more HMCD-DHA-bis-ether and one or more HMCD-DHA-mono-ester; c) one or more HMCD-DHA-bis-carbamate and one or more HMCD-DHA-mono-ester; d) one or more HMCD-DHA-bis-thiocarbamate and one or more HMCD-DHA-mono-ester; and wherein the dosage form further comprises one or more pharmaceutical excipient.

In embodiments, provided are methods wherein the schedule includes administration of a loading dose administered at least one or more hour prior to administration of one or more maintenance dose; wherein the loading dose consists of a separate dosage form that comprises one or more loading compound and one or more pharmaceutical excipient, and does not comprise the one or more maintenance compound; and wherein the one or more maintenance dose consists of a dosage form that comprises the one or more maintenance compound and one or more pharmaceutical excipient, and optionally comprises the loading compound; and wherein loading and maintenance compounds are thus administered sequentially in time, and selected from the following: a) a loading dose of one or more HMCD-DHA-mono-ether and a maintenance dose of one or more HMCD-DHA-mono ester; b) a loading dose of one or more HMCD-DHA-bis-ether and a maintenance dose of one or more HMCD-DHA-mono-ester; c) a loading dose of one or more HMCD-DHA-bis-carbamate and a maintenance dose of one or more HMCD-DHA-mono-ester; d) a loading dose of one or more HMCD-DHA-bis-thiocarbamate and a maintenance dose of one or more HMCD-DHA-mono-ester.

In embodiments, provided are HMCD dyes of formulae FV, FVI and FVII below:

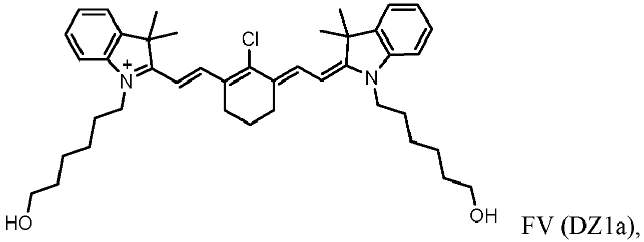

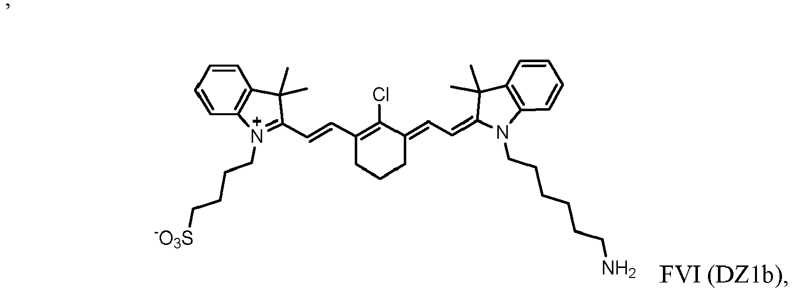

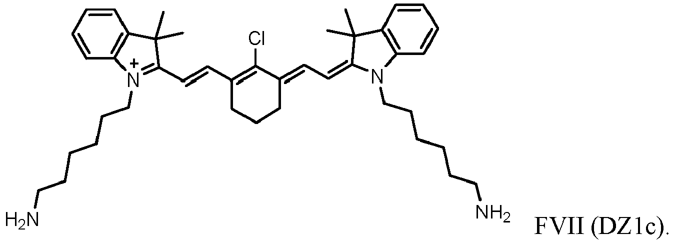

In embodiments, provided is a process of making a HMCD-drug conjugate wherein a HMCD is reacted with one or more further educts to form the conjugate, wherein the one or more further educts comprise a drug, or a derivative of the drug, and wherein the HMCD is selected from an HMCD of formulae FV, FVI and FVII (DZ1a, DZ1b and DZ1c) as shown herein.

In embodiments, provided is a process of making a a HMCD-drug conjugate wherein the conjugate is a THAD, wherein a HMCD is reacted with one or more further educts to form the THAD, wherein the one or more further educts comprise artemisinin or dihydroartemisinin (DHA), or a derivative of artemisinin or dihydroartemisinin (DHA), and wherein the HMCD is selected from an HMCD of formulae FV, FVI and FVII (DZ1a, DZ1b and DZ1c) as shown herein.

In embodiments, provided is a DRG-HMCD drug-dye conjugate wherein the HMCD residue is selected from the group consisting of DZ1a, DZ1b and DZ1c, wherein the drug and the HMCD are liked by ether, ester, carbamate or thiocarbamate linkage, wherein the linkage may be a mono-linkage to one drug molecule, or a bis-linkage to two drug molecules, and wherein the DRG-HMCD is selected from the group comprising the following conjugate types: a) DZ1a-DRG-ether, DZ1a-bis-DRG-ether, DZ1a-DRG-ester, DZ1a-bis-DRG-ester, DZ1a-DRG-carbamate, DZ1a-bis-DRG-carbamate, DZ1a-DRG-thiocarbamate, DZ1a-bis-DRG-thiocarbamate; b) DZ1b-DRG-ether, DZ1b-bis-DRG-ether, DZ1b-DRG-ester, DZ1b-bis-DRG-ester, DZ1b-DRG-carbamate, DZ1b-bis-DRG-carbamate, DZ1b-DRG-thiocarbamate, DZ1b-bis-DRG-thiocarbamate; and c) DZ1c-DRG-ether, DZ1c-bis-DRG-ether, DZ1c-DRG-ester, DZ1c-bis-DRG-ester, DZ1c-DRG-carbamate, DZ1c-bis-DRG-carbamate, DZ1c-DRG-thiocarbamate, DZ1c-bis-DRG-thiocarbamate.

In embodiments, provided is a DRG-HMCD drug-dye conjugate wherein the conjugated drug (DRG) is a residue of DHA or artemisinin, or a derivative of DHA or artemisinin.

In embodiments, provided is a THAD selected from the group consisting of a THAD as shown in the formulae below below:

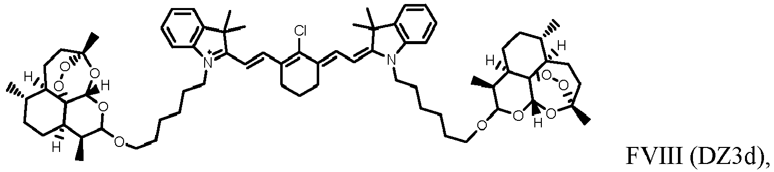

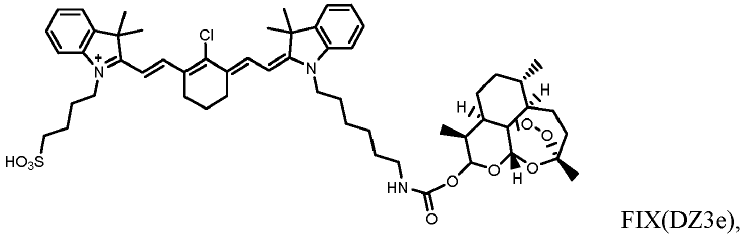

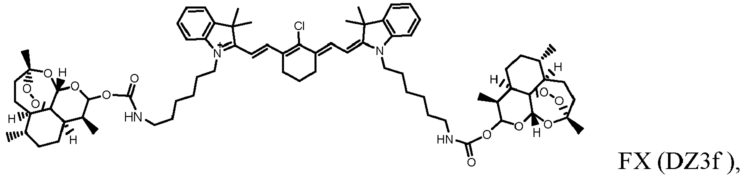

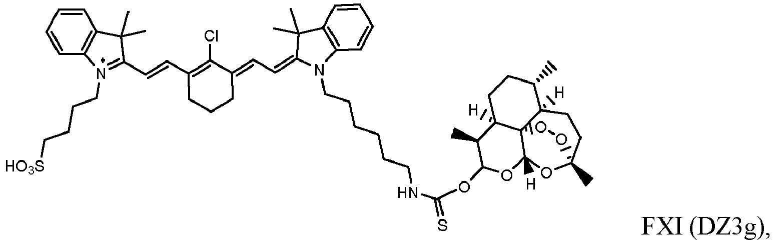

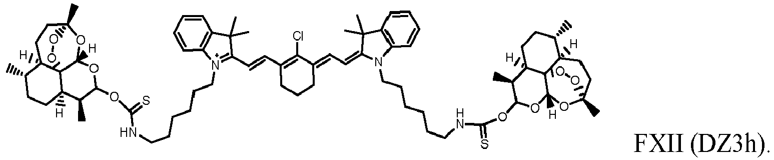

In embodiments, DHA derivatives may comprise two DHA moieties linked to the HMCD dye ("bis-DHA-derivatives"). The bis-DHA derivatives include bis-ether-derivatives, bis-ester derivatives, bis-carbamate derivatives and bis-thiocarbamate derivatives, and mixed derivatives, e.g. one DHA moiety is linked via an ether bond, and the other DHA moiety is linked via an ester bond (i.e. a bis-DHA-ether-ester, or for other combinations: a bis-DHA-ether-carbamate, bis-DHA-ether-thiocarbamate, bis-DHA-ester-carbamate, bis-DHA-ester-thiocarbamate, and bis-DHA-carbamate-thiocarbamate). Without wishing to be bound by theory, these bis-DHA derivatives are believed to have superior effects, including an improved dose response and growth inhibition of cancer cells and/or other improved anti-cancer effects.

In embodiments, THAD are provided wherein the A⁻ group may be selected from the group comprising I⁻, Cl⁻, Br⁻, OSO₂R⁻, BF₄⁻, ClO₄⁻.

In embodiments, THAD of formula FI, FII, FIII or FIV are provided wherein R₁ = (CH₂)₄-SO₃⁻ and X=Cl. Further, THAD of formula FI, FII, FIII or FIV are provided wherein R₁ = (CH₂)₄-SO₃⁻ , X=Cl, and R₁/R₂ are independently selected as indicated below.

| X*** | R₃ | R₁/R₂** |
|---|---|---|
| Halogen | Methyl | H, EDG, EWG |
| Halogen | Ethyl | H, EDG, EWG |
| Halogen | Propyl | H, EDG, EWG |
| Halogen | Butyl* | H, EDG, EWG |
| Halogen | Pentyl* | H, EDG, EWG |
| Halogen | Hexyl* | H, EDG, EWG |
| Halogen | Heptyl* | H, EDG, EWG |
| Halogen | Octyl* | H, EDG, EWG |
| Halogen | Nonyl* | H, EDG, EWG |
| Halogen | Decyl* | H, EDG, EWG |
| Halogen | Undecyl* | H, EDG, EWG |
| Halogen | Dodecyl* | H, EDG, EWG |
| Halogen | Tridecyl* | H, EDG, EWG |
| Halogen | Tetradecyl* | H, EDG, EWG |
| Halogen | Pentadecyl* | H, EDG, EWG |
| Halogen | Hexadecyl* | H, EDG, EWG |
| Halogen | Heptadecyl* | H, EDG, EWG |
| Halogen | Octadecyl* | H, EDG, EWG |
| Halogen | CH₂-SO₃⁻ | H, EDG, EWG |
| Halogen | (CH₂)₂-SO₃⁻ | H, EDG, EWG |
| Halogen | (CH₂)₃-SO₃⁻ | H, EDG, EWG |
| Halogen | (CH₂)₄-SO₃⁻ | H, EDG, EWG |
| Halogen | (CH₂)₅-SO₃⁻ | H, EDG, EWG |
| Halogen | (CH₂)₆-SO₃⁻ | H, EDG, EWG |
| Halogen | (CH₂)₇-SO₃⁻ | H, EDG, EWG |
| Halogen | (CH₂)₈-SO₃⁻ | H, EDG, EWG |
| Halogen | (CH₂)₉-SO₃⁻ | H, EDG, EWG |
| Halogen | (CH₂)₁₀-SO₃⁻ | H, EDG, EWG |
| Halogen | (CH₂)₁₁-SO₃⁻ | H, EDG, EWG |
| Halogen | (CH₂)₁₂-SO₃⁻ | H, EDG, EWG |
| Halogen | (CH₂)₁₃-SO₃⁻ | H, EDG, EWG |
| Halogen | (CH₂)₁₄-SO₃⁻ | H, EDG, EWG |
| Halogen | (CH₂)₁₅-SO₃⁻ | H, EDG, EWG |
| Halogen | (CH₂)₁₆-SO₃⁻ | H, EDG, EWG |
| Halogen | (CH₂)₁₇-SO₃⁻ | H, EDG, EWG |
| Halogen | (CH₂)₁₈-SO₃⁻ | H, EDG, EWG |
| Halogen | CH₂-CO₂⁻ | H, EDG, EWG |
| Halogen | (CH₂)₂-CO₂⁻ | H, EDG, EWG |
| Halogen | (CH₂)₃-CO₂⁻ | H, EDG, EWG |
| Halogen | (CH₂)₄-CO₂⁻ | H, EDG, EWG |
| Halogen | (CH₂)₅-CO₂⁻ | H, EDG, EWG |
| Halogen | (CH₂)₆-CO₂⁻ | H, EDG, EWG |
| Halogen | (CH₂)₇-CO₂⁻ | H, EDG, EWG |
| Halogen | (CH₂)₈-CO₂⁻ | H, EDG, EWG |
| Halogen | (CH₂)₉-CO₂⁻ | H, EDG, EWG |
| Halogen | (CH₂)₁₀-CO₂⁻ | H, EDG, EWG |
| Halogen | (CH₂)₁₁-CO₂⁻ | H, EDG, EWG |
| Halogen | (CH₂)₁₂-CO₂⁻ | H, EDG, EWG |
| Halogen | (CH₂)₁₃-CO₂⁻ | H, EDG, EWG |
| Halogen | (CH₂)₁₄-CO₂⁻ | H, EDG, EWG |
| Halogen | (CH₂)₁₅-CO₂⁻ | H, EDG, EWG |
| Halogen | (CH₂)₁₆-CO₂⁻ | H, EDG, EWG |
| Halogen | (CH₂)₁₇-CO₂⁻ | H, EDG, EWG |
| Halogen | (CH₂)₁₈-CO₂⁻ | H, EDG, EWG |
| Halogen | CH₂-NH₂ | H, EDG, EWG |
| Halogen | (CH₂)₂- NH₂ | H, EDG, EWG |
| Halogen | (CH₂)₃- NH₂ | H, EDG, EWG |
| Halogen | (CH₂)₄- NH₂ | H, EDG, EWG |
| Halogen | (CH₂)₅- NH₂ | H, EDG, EWG |
| Halogen | (CH₂)₆- NH₂ | H, EDG, EWG |
| Halogen | (CH₂)₇- NH₂ | H, EDG, EWG |
| Halogen | (CH₂)₈- NH₂ | H, EDG, EWG |
| Halogen | (CH₂)₉- NH₂ | H, EDG, EWG |
| Halogen | (CH₂)₁₀-NH₂ | H, EDG, EWG |
| Halogen | (CH₂)₁₁-NH₂ | H, EDG, EWG |
| Halogen | (CH₂)₁₂-NH₂ | H, EDG, EWG |
| Halogen | (CH₂)₁₃-NH₂ | H, EDG, EWG |
| Halogen | (CH₂)₁₄-NH₂ | H, EDG, EWG |
| Halogen | (CH₂)₁₅-NH₂ | H, EDG, EWG |
| Halogen | (CH₂)₁₆-NH₂ | H, EDG, EWG |
| Halogen | (CH₂)₁₇-NH₂ | H, EDG, EWG |
| Halogen | (CH₂)₁₈-NH₂ | H, EDG, EWG |

| | | |
|---|---|---|
| * Each alkyl chain may optionally be branched, and the branch may constitute one or more of an alkyl chain, aryl ring, heteroaryl group, aralkyl group; one or more positions of the chain or of a branch may be unsaturated. ** The R₁ and R₂ group may be independently selected from H, an electron withdrawing group (EWG), or an electron donating group (EDG). Example R₁/R₂ groups are indicated in the table further below. *** Halogen may be independently selected from bromine, chlorine, fluorine and iodine. | | |

In embodiments, THAD of formula FI, FII, FIII or FIV are provided wherein X and R₃ are selected as indicated herein above, and R₁/R₂ are independently selected as indicated in the table below.

| R₁/R₂ |
|---|
| H |
| OCH₃ |
| SCH₃ |
| NH₂ |
| NHCH₃ |
| N(CH₃)₂ |
| NHCOCH₃ |
| SH |
| OH |
| F |
| Cl |
| Br |
| I |
| CH₃ |
| CH₂CH₃ |
| (CH₂)₂CH₃ |
| NO₂ |
| CN |
| COOH |
| COOCH₃ |
| COOCH₂CH₃ |
| CF₃ |
| CCl₃ |
| SO₃H |
| PO₃H |

DHA and its derivatives may have side effects, which may be more pronounced or occur more frequently when the drug is used in combination therapy together with other drugs. Reported side effects include, among others, nausea, vomiting, anorexia, dizziness, blood abnormalities, allergic reaction, liver inflammation, and effects on the auditory and vestibular system (e.g. tinnitus and subclinical hearing loss). Without wishing to be bound by theory, the targeted delivery and lower concentrations of the THAD may allow to reduce or avoid one or more of these side effects.

Without wishing to be bound by theory, it appears that certain DHA conjugates, in particular monoester conjugates, may contribute to organ abnormalities such as liver abnormalities, and possibly liver toxicity; such effects may be avoided by some or all THAD as described herein.

A particular group of THAD and their derivatives may include conjugates that are ether conjugates, in particular monoether conjugates. Without wishing to be bound by theory, the efficacy of these conjugates may be higher than e.g. that of monoester conjugates; due to their higher efficacy at a lower non-toxic dose these compounds may allow to avoid toxicity, including e.g. liver and/or kidney toxicity.

Similarly, another particular group of THAD and their derivatives may include conjugates that are bis-ether conjugates. Without wishing to be bound by theory, the efficacy of these conjugates may be higher than e.g. that of monoester and/or monoether conjugates; due to their higher efficacy at a lower non-toxic dose these compounds may allow to avoid toxicity, including e.g. liver and/or kidney toxicity.

Yet another particular group of THAD and their derivatives may include mono conjugates wherein R₃ is (CH₂)ₙ-SO₃⁻, wherein n is independently selected from the group consisting of 1, 2, 3, 4, 5, 6, 7, 8, 9, 10, 11, 12, 13, 14, 15, 16, 17 ,18, 19, 20; in particular, n may be 2-6, more particularly 4. Without wishing to be bound by theory, such mono-conjugates may provide a decreased toxicity, for example liver and/or kidney toxicity, e.g. in comparison to conjugates without a sulfonic acid group.

Still another group of particular THAD and their derivatives may include conjugates that are monoether conjugates, wherein R₃ is (CH₂)ₙ-SO₃⁻, and wherein n is 1-20, as detailed above. Without wishing to be bound by theory, these monoether conjugates may provide a decreased toxicity, for example liver and/or kidney toxicity, e.g. in comparison to monoether conjugates without a sulfonic acid group, and at the same time may avoid toxicity due to their higher efficacy at a lower non-toxic dose, e.g. compared to the monoester.

Without wishing to be bound by theory, the THAD and groups of THAD described herein may be useful for substantially non-toxic low-dose treatments in various cancers, particularly certain types of cancers, including, e.g. without limitation, kidney, prostate, pancreatic, and lung cancers, and in particular the various aggressive forms thereof. Suitable doses may be as low as about 0.25-10 mg/kg or lower, e.g. about 0.5-6 mg/kg or 1-3 mg/kg, more particularly about 1-2 mg/kg.

In embodiments, the growth inhibition of cancer cells and other anti-cancer effects provided by the THAD may include one or more of anti-proliferative and anti-angiogenic effects, induction of apoptosis, oxidative stress, inhibition of oncogenes, and activation of tumor suppressor genes, suppressing the cells proliferation, inducing apoptotic response, arresting tumor cell cycle, inhibiting cells invasion, inhibiting metastasis, preventing angiogenesis, altering oxidative damage reactions, disrupting cancer signaling pathways, regulating tumor microenvironment, and activating immune response to cancer cells.

THAD may be formed as follows. To form the lactol DHA, for example, the lactone of artemisinin can selectively be reduced with mild hydride-reducing agents such as sodium borohydride, potassium borohydride, or lithium borohydride. Derivatives of DHA comprising one or more substituents may also be useful. Particularly useful DHA derivatives retain the peroxide bridge in its endoperoxide 1,2,4-trioxane ring. Without wishing to be bound by theory, this endoperoxide is believed to substantially contribute to DHA's mechanism of action in conjugated form and/or after its release, if any.

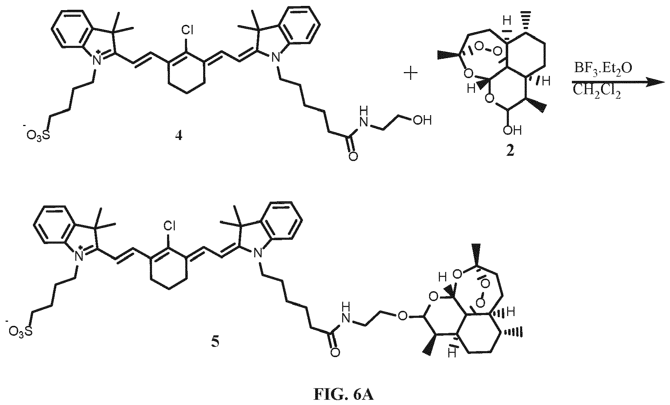

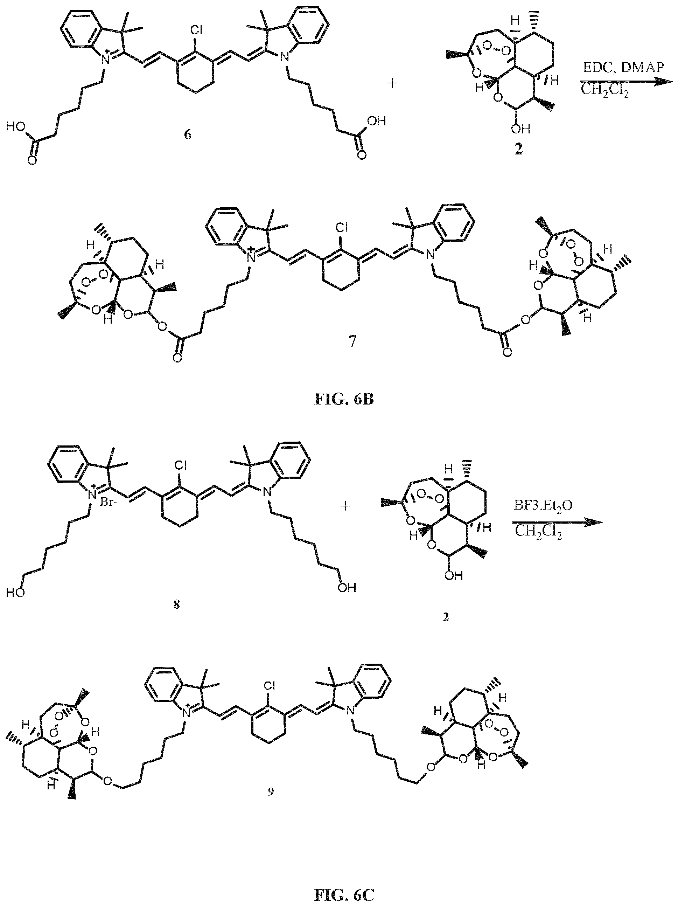

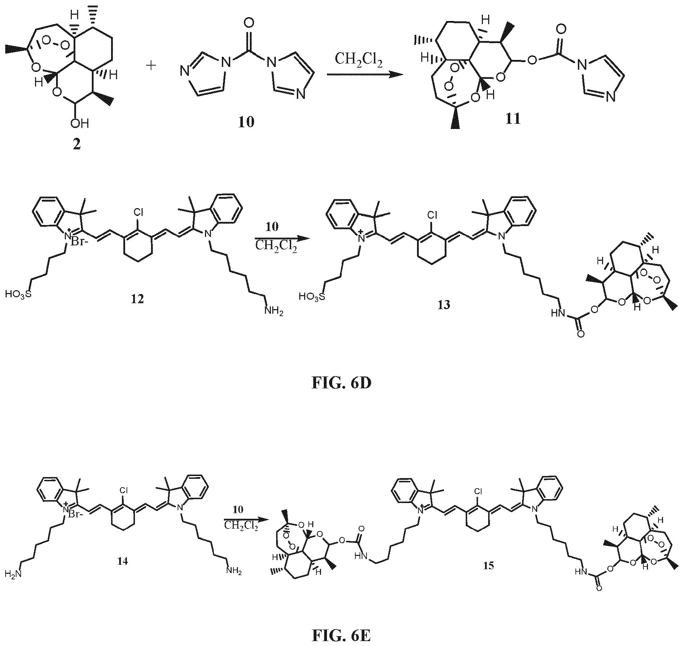

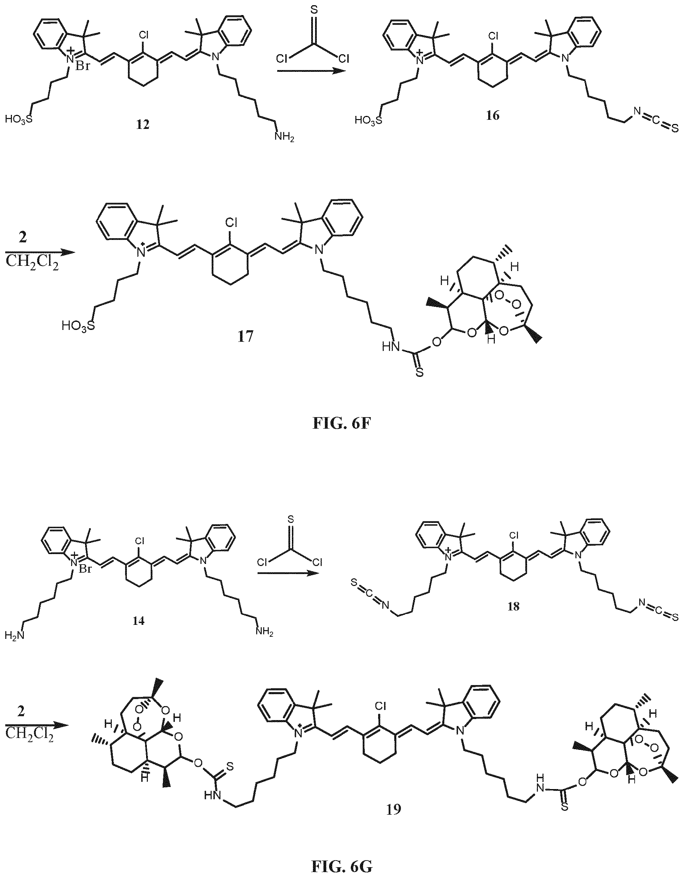

An illustrative synthesis is described in detail the examples (e.g. for the mono-ether DZ1-DHA conjugate and the mono-ester DZ1-DHA conjugate), and illustrative reaction schemes for synthesis for a DZ1-DHA-ether, MHI-148 bis-DHA-ester, DZ1a-bis-DHA-ether, DZ1b-DHA-carbamate, DZ1c-bis-DHA-carbamate, DZ1b-DHA-thiocarbamate, DZ1c-bis-DHA-thiocarbamate respectively, are shown in **FIG. 6A****,** **FIG. 6B****,** **FIG. 6C****,** **FIG. 6D****,** **FIG. 6E****,** **FIG. 6F** and **FIG. 6G****.** These may be adapted accordingly to form e.g. the bis-ether, bis-ester or bis-carbamate/thiocarbamate, as will be apparent to the skilled person, e.g. starting from DZ1a, MHI-148, DZ1b or DZ1c and forming a DZ1a-148-bis-DHA-ether, MHI-148-bis-DHA-ester, DZ1b-mono-DHA-carbamate/thiocarbamate or DZ1c-bis-DHA-carbamate/thiocarbamate.

An illustrative list of educts (MHI148, DZ1, DZ1a, DZ1b, DZ1c) and resulting conjugates (DZ3a, b, c, d, e, f, g, and h) are shown in the overview table below. Other suitable methods and corresponding materials to make the various THAD are known and may be used accordingly, depending on the desired THAD and its desired HMCD-DHA linkage (e.g. bi-ester, bi-carbamate/thiocarbamate); accordingly the proper HMCD can be used as an educt, and the reaction type and further educt(s) can be chosen accordingly to form the desired linkage, as will be apparent to a person of ordinary skill in the art.

| **Structure** | **Name (type)** | **Cpd #** |
|---|---|---|
| 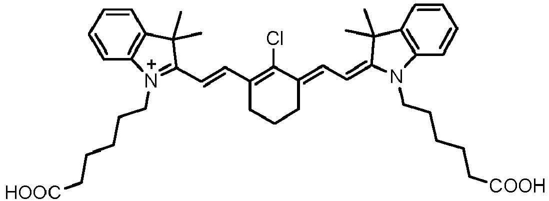 | MHI148 | Cpd. 6 |
| 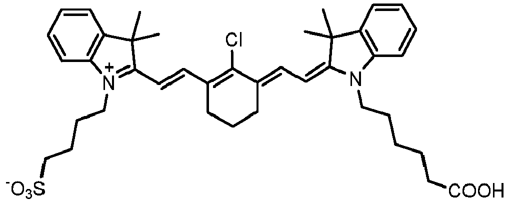 | DZ1 | n/a |
| 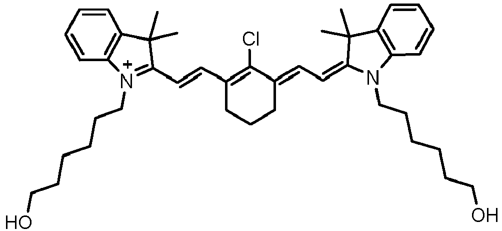 | DZ1a | Cpd. 8 |
| 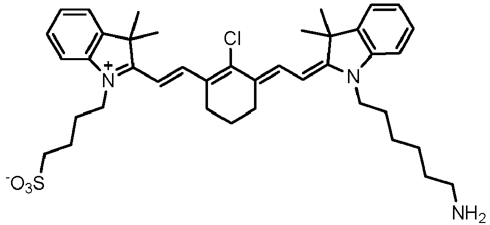 | DZ1b | Cpd. 12 |
| 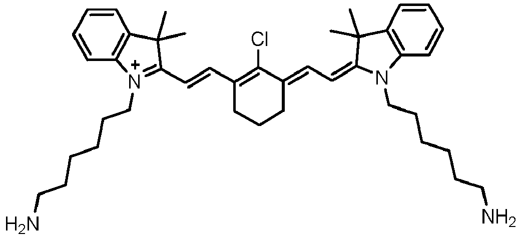 | DZ1c | Cpd. 14 |
| 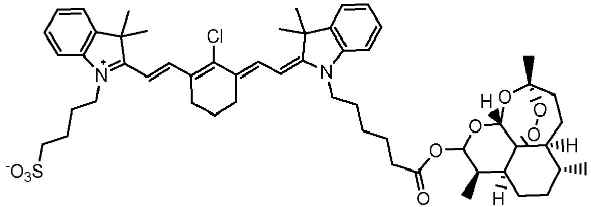 | DZ3a (DZ1-DHA-ester) | n/a |
| 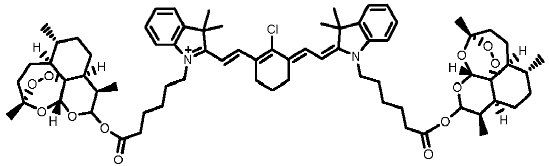 | DZ3b (MHI148-bis-DHA ester) | Cpd. 7 |
| 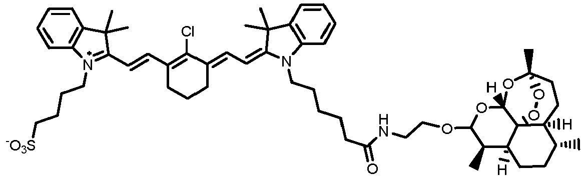 | DZ3c (DZ1-DHA-ether) | Cpd. 5 |
| 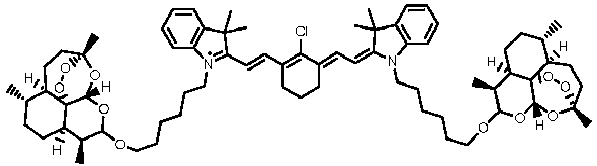 | DZ3d (DZ1a-bis-DHA-ether) | Cpd. 9 |
| 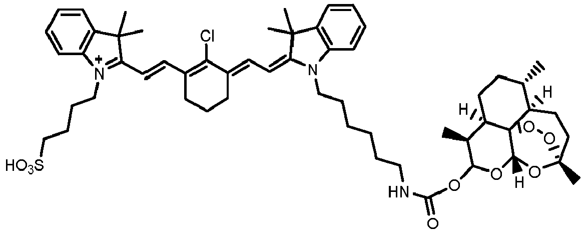 | DZ3e (DZ1b-DHA-carbamate) | Cpd, 13 |
| 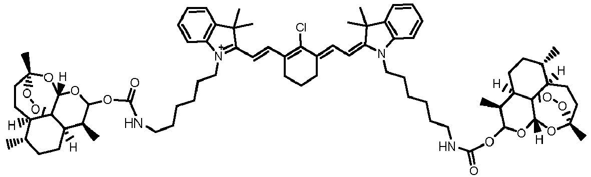 | DZ3f (DZ1c-bis-DHA-carbamate) | Cpd. 15 |
| 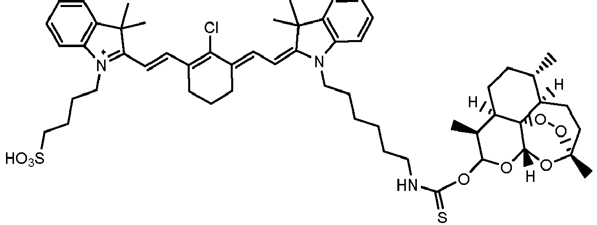 | DZ3g (DZ1b-DHA-thiocarbamate) | Cpd. 17 |
| 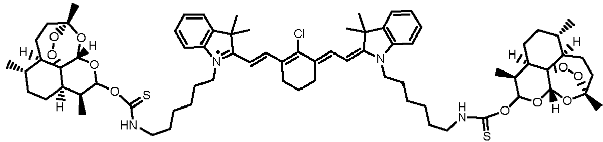 | DZ3h (DZ1c- bis-DHA-thiocarbamate) | Cpd. 19 |

In embodiments, one or more THAD may be administered to patients or patient groups at elevated risk for development of tumors or cancer, to lower their risk to develop cancers or tumors, or to slow the growth of existing tumors or prevent existing tumors from growing and/or spreading. These may include patients/patient groups who have or are at increased risk for cancer (in particular e.g. prostate cancer, kidney cancer, lung cancer and breast cancer), as determined by biomarkers, genetic screening or risk profile, including e.g. environmental risks, life style, family history, and/or detection of potential pre-cancerous lesions (e.g. breast nodules).

For prostate cancer such environmental risks may include smoking, obesity, diet (e.g. high levels of calcium) and aberrations or mutations in certain genes (e.g. RNASEL, formerly known as HPCI, BRCA1 and BRCA2, which have also been linked to breast and ovarian cancer in women, MSH2, MLH1, and other DNA mismatch repair genes, HOXB13). For pancreatic cancer these may include smoking, exposure to mutagenic nitrosamines, organ-chlorinated compounds, heavy metals, and ionizing radiations, chronic pancreatitis, alcohol, microbial infections, obesity, diabetes, gallstones and/or cholecystectomy, accumulation of asbestos fibers, and aberrations or mutations in certain genes (e.g. BRCA1, BRCA2, PALB2). For kidney cancer these may include smoking and other exposure to hazardous substances, such as arsenic, asbestos, cadmium, some herbicides, benzene, and trichloroethylene (TCE), family history, and aberrations or mutations in certain genes (e.g. MET oncogene, VHL, FH, FLCN, SDHB, SDHD, fumarate hydratase, succinate dehydrogenase, TSC1, TSC2, and TFE3 genes). For lung cancer these include smoking, exposure to radon, chemicals, asbestos and dust, family history and aberrations or mutations in certain genes (e.g. ROS1, RET proto-oncogene, BRAF).

In embodiments, methods for treating cancer or decreasing future risk of cancer (for example by, e.g., improving mitochondrial or lysosomal function, such as, e.g., lowering the mitochondrial oxygen consumption rate (OCR), improving the extracellular acidification rate ("ECAR"), and reducing or preventing the removal of polyubiquinated proteins) are provided.

In embodiments, THAD and methods described herein may be suitable for treatment or risk reduction relating to one or more of the following cancers: prostate cancer, pancreatic cancer, lung cancer, NSCLC (non-small cell lung carcinoma), SCLC (small cell lung carcinoma), kidney cancer, lymphoma, colorectal cancer, skin cancer, HCC cancer, and breast cancer, squamous-cell carcinoma of the lung, anal cancers, glioblastoma, epithelial tumors of the head and neck, and other cancers. Non-small-cell lung carcinoma may include Squamous-cell carcinoma, Adenocarcinoma (Mucinous cystadenocarcinoma), Large-cell lung carcinoma, Rhabdoid carcinoma, Sarcomatoid carcinoma, Carcinoid, Salivary gland-like carcinoma, Adenosquamous carcinoma, Papillary adenocarcinoma, and Giant-cell carcinoma. Small-cell lung carcinoma may include Combined small-cell carcinoma. Non-carcinoma of the lung may include Sarcoma, Lymphoma, Immature teratoma, and Melanoma. Without wishing to be bound by theory, it is believed that the THAD have broad applicability for different types of cancers, tumors, and their metastases.

In embodiments, THAD may be particularly beneficial to treat patients with prostate, kidney, pancreatic or lung cancer, or patients at higher than average risk to develop such cancers.

In embodiments, THAD may be particularly beneficial to treat patients with lung cancer, or patients at higher than average risk to develop lung cancer (e.g. smokers). Lung cancers may include the two main types of cancer i.e. non-small cell lung cancers (NSCLC) and small cell lung cancer. The most common lung cancer is adenocarcinoma of the lung (AC) which is one of the three lung cancers of the NSCLC type (the other two NSCLC cancers being squamous cell carcinoma and large cell carcinoma). In contrast to small cell lung cancer (SCLC), NSCL incl. AC typically respond to various milder non-chemotherapeutic treatment options, including TKI. SCLC is the form most strongly related to cigarette smoking and is more difficult to treat, typically requires chemotherapy, and is prone to develop resistance upon chemotherapy administration. THAD may allow treatment of patients despite them having acquired resistance (e.g. against TKI and/or chemotherapeutics), or may avoid development of resistance that occurs in these cancers upon treatment altogether.

In embodiments, THAD may be administered to a patient presenting with a brain tumor or brain metastase. The THAD may cross the blood-brain barrier (BBB), thus it may provide its anti-cancer effects to brain tumors or brain metastases of various cancers, in particular of the cancers as described herein-above, which may be treated according to the methods of administration as described herein, including in particular systemic administration methods.

In embodiments, a pharmaceutical composition comprising one or more THAD as an active is provided. The pharmaceutical composition may be for human or for veterinary use, and comprise one or more compound of the invention (or a salt, solvate, metabolite, or derivative thereof) with one or more pharmaceutically acceptable carrier and/or one or more excipient and/or one or more active. The one or more carrier, excipient and/or active may be selected for compatibility with the other ingredients of the formulation and not unduly deleterious to the recipient thereof. Such carriers are known in the art and may be selected as will be apparent to a person of ordinary skill in the art.

In embodiments, pharmaceutical kits are provided. Such kits can comprise one or more THAD or composition comprising a THAD, preferably in form of its salt, and, typically, a pharmaceutically acceptable carrier. The kit can also further comprise conventional kit components, such as needles for use in injecting the composition(s), one or more vials for mixing the composition components, and the like, as are apparent to those of ordinary skill in the art. In addition, instructions, e.g. as inserts or as labels, indicating quantities of the components, guidelines for mixing the components, and protocols for administration/co-administration, can be included in the kit. In particular, the kit may comprise instructions for a co-administration schedule of the one or more THAD and optional drugs as described herein below.

In embodiments, routes of administration for the one or more THAD and pharmaceutical compositions comprising it may be systemic (administration into the circulatory system so that the entire body is affected) or local/tissue-specific, and may be include, but are not limited to: oral, intraperitoneal, subcutaneous, intramuscular, transdermal, rectal, vaginal, sublingual, intravenous, buccal, topical, transdermal, by implantation, by inhalation, and by skin patch. In some embodiments, the pharmaceutical compositions of the invention contain a pharmaceutically acceptable excipient suitable for rendering the compound or mixture administrable via the above routes of administration.

Advantageously, in embodiments, the THAD may be administered to patients at a low frequency, avoiding the requirement of multiple daily or daily dosing. Depending on the amount, for example, doses may be administered e.g. every 2, 3, 4, 5, 6, or 7 days, or at longer intervals. Preferably, administration may be once a week. Even longer intervals such as once every 2, 3 or 4 weeks may be possible depending on the amount of THAD administered with each dose and individual patient requirements, including e.g. the degree of desired anti-cancer effects, the patient's cancer type and rapidity of tumor growth or/or spreading of metastases, and the level of side effects considered acceptable. Without wishing to be bound by theory, it is believed this is due to tight HMCD binding of this new DHA derivative, which keeps it in the cancer cells for extended periods of time for multiple days, likely weeks or months, thus providing prolonged anti-cancer activity while preventing side effects.

Without wishing to be bound by theory, it is believed that THAD may have a similar or better growth inhibitory effect on tumors *in vivo* compared to tyrosine kinase inhibitors (TKI). Thus, advantageoulsy, THAD may be administered to patients who were administered TKI, which are often associated with rapid development of drug resistance by the TKI-treated cancer cells; alternatively, THAD may be administered instead of a TKI (thus avoiding development of resistance while providing a similar or better growth inhibitory effect compared to the TKI), to patient groups typically benefiting from administration of TKI.

A TKI is an agent or pharmaceutical drug that inhibits tyrosine kinases. Tyrosine kinases are enzymes responsible for the activation of many proteins by signal transduction cascades, including in particular EGFR (EGFR-TKI), ALK (ALK-TKI), and ROS1 (ROS-TKI). For example, the proteins are activated by adding a phosphate group to the protein (phosphorylation), a step that the TKI inhibits. TKI are typically used as anticancer drugs against various cancers to inhibit the growth of the cancer cells (and stop or slow down tumor growth), and/or to induce the cells to undergo apoptosis (cell death), typically resulting in tumor shrinkage. Gene rearrangement events involving the relevant tyrosine kinase receptor gene, e.g. the EGFR, ALK, and ROS1 gene, have been described to occur in various cancers, including lung cancers. Various cancers and tumors, including of the lung (e.g. NSCLC, NSCLC/AC), were found to be responsive to TKI, including one or more of EGFR-TKI, ALK-TKI, and ROS-1-TKI. In all TKI, the emergence of resistance upon TKI administration to cancer patients is common.

TKI, including in particular EGFR-TKI, are used treat various cancers including, in particular, without limitation, non-small cell lung carcinoma (NSCLC).

Epidermal growth factor receptor (EGFR) is a member of the ErbB family of receptors, a subfamily of four closely related receptor tyrosine kinases: EGFR (ErbB-1), HER2/neu (ErbB-2), Her 3 (ErbB-3) and Her 4 (ErbB-4). Mutations affecting EGFR expression or activity may result in many types of cancer, including, e.g., NSCLC, adenocarcinoma of the lung (AC), anal cancers, glioblastoma, epithelial tumors of the head and neck; Cancer-causing mutations include e.g. EGFRvIII (e.g. glioblastoma), other aberrations include amplification or dysregulation. The main activating EGFR-mutations include, without limitation, L858R mutation, deletions (Del) in exon 19 (Del19), and T790M; further mutations include, e.g., E746-A750 deletion, L747-E749 deletion, A750P mutation, and C797S mutation, among others. MET amplification is another mechanism of resistance to both EGFR-TKIs including e.g. AZD9291 and CO1686.

Anaplastic lymphoma kinase (ALK) also known as ALK tyrosine kinase receptor is an enzyme that in humans is encoded by the ALK gene. ALK aberrations play a role in cancers including, e.g., anaplastic large-cell lymphoma, NSCLC, adenocarcinoma of the lung (AC), neuroblastoma, inflammatory myofibroblastic tumor, renal cell carcinomas, esophageal squamous cell carcinoma, breast cancer (in particular the inflammatory subtype), colonic adenocarcinoma, glioblastoma multiforme, and anaplastic thyroid cancer, among others.

Proto-oncogene tyrosine-protein kinase (ROS or ROS1) is an enzyme that in humans is encoded by the ROS1 gene with structural similarity to the ALK protein. ROS is a receptor tyrosine kinase with structural similarity to the anaplastic lymphoma kinase (ALK) protein. Gene rearrangement events involving ROS1 have been described in lung and other cancers, and such tumors have been found to be responsive to TKI.

THAD may be administered to cancer patients, in particular to cancer patient groups that respond to, or are likely to respond to, treatment with tyrosine kinase inhibitors (TKI), based on e.g. tumor type/grading, tumor histology, prior treatment, resistance to one or more TKI, or various biomarkers, including mutations or aberrations in one or more gene that effects the activity of cellular tyrosine kinase receptors (including, e.g., EGFR, ALK, ROS1 and BRAF). Such patient groups are typically treated with the corresponding inhibitor(s), including, e.g., EGFR-TKI, ALK-TKI, ROS-TKI, and BRAF-TKI. Such patient groups include those with, for example, without limitation, non-small cell lung cancer (NSCLC), and in particular NSCLC with adenocarcinoma (AC), which are common types of lung cancer with increasing incidence. A subgroup of NSCLC patients harbors a particular oncogenic driver, namely activating EGFR, ALK-, or ROS1-aberrations (including chromosomal rearrangements, translocations, or mutations); and these oncogenic drivers appear to be almost exclusively present in AC.

EGFR-TKI include, for example, without limitation: Gefitinib, Icotinib, Erlotinib, Brigatinib, Dacomitinib, Lapatinib, Vandetanib, Afatinib, Osimertinib (AZD9291), CO-1686, HM61713, Nazartinib (EGF816), Olmutinib, PF-06747775, YH5448, Avitinib (AC0010), Rociletinib, and Cetuximab; they are used to treat various cancers including in particular lung cancer, NSCLC, colon cancer, metastatic colorectal cancer, and head and neck cancer, among others.

Based on their mechanism, EGFR-TKI can be grouped into three groups: 1st, 2nd or 3rd generation TKI/EGFR-TKI. Examples for 1st generation EGFR-TKI include, e.g., Gefitinib, Icotinib, and Erlotinib. Examples for 2nd generation EGFR-TKI include, e.g., Afatinib and Dacomitinib. 2nd generation EGFR-TKI typically irreversibly bind to the tyrosine kinase of EGFR and other ErbB-family members. Uses for 2nd generation EGFR-TKI include, e.g., first-line treatment of advanced NSCLC harboring activating EGFR mutations. Examples for 3rd generation EGFR-TKI include, e.g., Osimertinib (AZD9291), CO-1686, HM61713, Nazartinib (EGF816), Olmutinib, PF-06747775, YH5448, Afatinib Avitinib (AC0010), and Rociletinib.

All generations of TKI exhibit a tendency for the treated tumor/cancer cells to develop resistance, with 2nd and 3rd generation drugs typically used to treat cancers that harbor mutations in the relevant gene, in particular the epidermal growth factor receptor (EGFR) gene, and/or display resistance to a 1st generation TKI. The grouping is based on mechanism and correspondingly patient group that may best benefit from the drug, and may also determine the risk of developing resistance, e.g. developing one or more of various mutations, in particular EGFR mutations, or mutations that allow to bypass the EGFR-related mechanism. 1st generation EGFR-TKI are effective, e.g., as first-line treatment of advanced NSCLC harboring activating EGFR mutations (deletions in exon 19 (Del19), and exon 21 L858R mutation); further mutations including in particular EGFR T790M resistance mutation (EGFR T790M) emerged in a large number of these patients. The 2nd and 3rd generation EGFR-TKI are designed for improvements over the 1st generation drugs, and in particular, to more potently inhibit EGFR and/or to overcome various mutations developed by patients, in particular after 1st generation treatment, such as EGFR T790M.

Without wishing to be bound by theory, it is believed that THAD may be able to circumvent TKI resistance and provide anti-cancer effects such as growth inhibition; thus THAD may be effective in patient groups that exhibit such resistance, in particular, patients and patient groups having undergone therapy with a TKI inhibitor, including a TKI inhibitor of the 1st, 2nd or third generation, and in particular patients suitable for or having undergone therapy with a 3rd generation TKI inhibitor, or with a tumor already TKI or 3rd generation TKI resistant.

Examples for 1st generation EGFR-TKI include Gefitinib and Erlotinib. Examples for 2nd generation EGFR-TKI include Afatinib and Dacomitinib. 2nd generation EGFR-TKI typically irreversibly bind to the tyrosine kinase of EGFR and other ErbB-family members. Uses for 2nd generation EGFR-TKI include first-line treatment of advanced NSCLC harboring activating EGFR mutations. 3rd generation EGFR-TKI may include, without limitation, one or more of: osimertinib (AZD9291), Rociletinib (CO-1686), HM61713, Nazartinib (EGF816), Olmutinib (HM61713), PF-06747775, YH5448, afatinib, avitinib (AC0010), and ASP8273. 3rd generation EGFR-TKI generally provide efficacy in patients with acquired resistance to 1st or 2nd-generation TKI. 3rd generation EGFR-TKI are typically EGFR-mutant selective and EGFR wild-type (WT) sparing, i.e. their activity against EGFR mutant cells is greater than against EGFR wildtype (WT) cells, e.g. at least 10, 100, 200 times or more greater activity. Also, 3rd generation EGFR-TKI are active against or inhibit both EGFR-activating and resistance mutations, in particular, e.g., the T790M resistance mutation. For example, 3rd generation EGFR-TKI such as Osimertinib, CO-1686, and HM61713 may selectively and irreversibly target both sensitizing/activating EGFR mutations and T790M resistance mutations, while sparing the wild-type EGFR tyrosine kinase.

Osimertinib is a mono-anilino-pyrimidine that selectively and irreversibly targets both sensitizing EGFR mutations and T790M resistance mutations, while sparing the wild-type EGFR tyrosine kinase. Specifically, Osimertinib is substantially less potent at inhibiting phosphorylation of EGFR in wild-type cell lines, e.g. about 100-200 times greater potency against L858R/T790M than wild-type EGFR, and used to treat cancers that developed resistance, or with a tendency to develop resistance, including in particular non-small cell lung carcinoma (NSCLC).

In embodiments, THAD may be administered as described herein to patients with cancers that have a tendency to develop resistance to TKI, to inhibit the growth of cancer cells while avoiding their development of resistance. Such cancers include advanced EGFR, ALK and/or ROS1 mutation-positive tumors, which are common, e.g., in non-small cell lung cancer (NSCLC). Thus, patient groups that may particularly benefit from THAD administration may include those with activating mutations of EGFR, ALF or ROS1 and/or resistance mutations of EGFR, ALF, or ROS1, including in particular acquired mutations during treatment with a TKI, EGFR-, ALF- or ROS-TKI, including a 1st, 2nd or 3rd generation TKI or EGFR-TKI. For example, patients with acquired T790M EGFR mutations are common among advanced NSCLC patients who progressed after first line EGFR TKI treatment, e.g. with a 1st or 2nd generation EGFR-TKI.

Specifically, THAD may be administered to patient groups typically treated with ALK-TKI (inhibitors to ALK tyrosine kinase receptor or CD246), to avoid development of resistance. Similar to other TKI, the emergence of resistance upon ALK-TKI is common. Also, THAD may be administered to patients after treatment with an ALK-TKI, to overcome acquired resistance to the ALK-TKI. Patient groups include those with cancers that are positive for genetic ALK aberrations, such as, e.g., metastatic NSCLC. Patient groups with ALK-positive cancers, in particular NSCLC, generally include non-smokers, those of younger age, adenocarcinoma histology, female gender and/or with pathological features that include a solid morphology and/or presence of signet ring cells.

ALK aberrations may include chromosomal rearrangements resulting in fusion genes, as seen, e.g., in ALCL and NSCLC. Other alterations include ALK copy-number gains and activating ALK mutations. Aberrations such as mutations or translocation of ALK are known to occur in various cancers, including, e.g., NSCLC, anaplastic large cell lymphomas (ALCL), inflammatory myofibroblastic tumors, diffuse large B cell lymphoma, colon cancer, renal cell carcinoma, breast carcinoma, esophageal cancer, and neuroblastoma.

Examples of ALK-TKI include, e.g., Brigatinib, Crizotinib, Ceritinib, Alectinib and Entrectinib (RXDX-101). Crizotinib (PF-02341066) is a 1st generation ALK-TKI and used e.g. for ALK-positive NSCLC and ROS 1-positive NSCLC, particularly locally advanced and/or metastatic NSCLC. It has an IC₅₀ against EML4-ALK of 250-300 nm. Ceritinib is used e.g. for ALK-positive metastatic NSCLC.

Similarly, THAD may be administered to patient groups generally treated with ROS-TKI (inhibitors to Proto-oncogene tyrosine-protein kinase ROS or ROS1). Patient groups include those with cancers that are positive for genetic ROS1 aberrations such as fusions or mutations, such as, e.g., metastatic NSCLC, and patients who developed resistance to treatment with a ROS-TKI. Cancers that may be positive for ROS1 aberrations include, e.g., without limitation: glioblastoma, lung cancers incl. lung adenocarcinoma, ovarian cancer, ovarian carcinoma, sarcoma, cholangiocarcinoma, cholangiosarcoma, inflammatory myofibroblastic cancer, gastric cancer, colorectal cancer, spitzoid melanoma, angiosarcoma, and others.

Examples of ROS-1 inhibitors include, e.g., Crizotinib, Entrectinib, Lorlatinib (PF-06463922), Ceritinib, TPX-0005, DS-6051b, and Cabozantinib. Crizotinib is used e.g. for metastatic ROS1-positive NSCLC). Cabozantinib is used e.g. for metastatic medullary thyroid cancer and renal cell carcinoma.

Some TKI are suitable to address multiple mechanisms of malignancy, and can be used for multiple patient groups, e.g. those that are EGFR-positive, ALK-positive, or ROS/ROS1-positive (EGFR+, ALK+, ROS+), i.e. that have genetic aberrations affecting these genes encoding the respective tyrosine kinase enzymes. For example, Entrectinib is a TKI for all of three Trk proteins (encoded by the three NTRK genes, respectively) as well as the ROS1, and ALK receptor tyrosine kinases. Similarly, Crizotinib inhibits both ALK and ROS1.

Without wishing to be bound by theory, it is believed that THAD may be able to provide similar or better effect than and/or circumvent resistance to one or more of the following: a hormonal antagonist, an anti-androgenic drug, Abiraterone, Enzalutamid, a chemotherapeutic drug, Docetaxel, Paclitaxel, and Cabazitaxel, and to provide anti-cancer effects such as growth inhibition; thus THAD may be effective in patient groups that exhibit resistance to one or more of such drugs, in particular, patients and patient groups having undergone therapy with these drugs, or having a tumor already resistant to one or more of these drugs.

In embodiments, the active ingredients (THAD, and optional secondary drug/active(s) such as chemotherapeutic or anti-androgenic) can be admixed or compounded with a conventional, pharmaceutically acceptable excipient. A mode of administration, vehicle, excipient or carrier should generally be substantially inert with respect to the actives, as will be understood by a person of ordinary skill in the art. Illustrative methods, vehicles, excipients, and carriers are described e.g. in Remington's Pharmaceutical Sciences, 18th ed. (1990), the disclosure of which is incorporated herein by reference. The excipient must be "acceptable" in the sense of being compatible with the other ingredients of the formulation and not deleterious to the recipient thereof.

In embodiments, in patients not yet having been administered cancer drugs that tend to induce drug resistance, and thus the risk of development of resistance may be lowered by co-administering a THAD together with the one or more cancer drug.

In embodiments, the THAD may be co-administered with further drugs in a coordinated administration schedule either concurrently or subsequently. Such drugs for co-administration with a THAD include in particular drugs that tend to induce drug resistance when administered on their own, for example, chemotherapeutics such as, e.g., TKIs, cisplatin and its derivatives, gemcitabine, and doxorubicin, hormonal antagonists, anti-androgenic drugs, such as, e.g., Abiraterone and Enzalutamide, and taxane drugs, such as, e.g., Docetaxel and Paclitaxel. Without wishing to be bound by theory, the combination of androgen deprivation and therapy with THAD may provide an additive or synergistic therapeutic effect.

In embodiments, taxanes (also known as taxoids) for co-administration are structurally a class of diterpenes that were originally identified from plants of the genus Taxus (yews) and are drugs used for chemotherapy; they comprise a taxadiene core and typically a 6/8/6 or 6/10/6-membered core ring. Taxanes may include one or more of Docetaxel (Taxotere), Paclitaxel (Taxol), Cabazitaxel. They also may include one or more abeotaxane, i.e. a class of taxoid molecules with an unconventional core 5/7/6 type ring structure, e.g., without limitation, taxchinin A. The core carbon skeleton of a conventional taxane has a 6-membered A ring, 8-membered B ring and a 6-membered C ring, combined with conventional side chains, while abeotaxanes contain three altered ring structures, with a 5-membered A ring, 7-membered B-ring and 6-membered C-ring (combined with conventional side chains). Other a 11 (15→1) abeotaxanes besides taxchinin A include brevifoliol and TPI 287 (formerly ARC-100). Other taxanes include taxchinin B (a 11 (15→1) obeotaxoid with an oxetane ring).

In embodiments, the pharmaceutical formulations may conveniently be made available in a unit dosage form by various methods well known in the pharmaceutical arts, for example by presenting the formulation in a suitable form for delivery, e.g., forming an aqueous suspension, compounding a tablet, or encapsulating a powder into a capsule, e.g. to release the powder at a particular time, stage or location of digestion, and/or protect it from stomach acids. The dosage form may optionally comprise one or more adjuvant or accessory pharmaceutical ingredient for use in the formulation, including, without limitation, mixtures, buffers, and solubility enhancers.

In embodiments, parenteral dosage formsinclude, but are not limited to, aqueous and non-aqueous sterile injection solutions, solutions ready for injection, dry products ready to be dissolved or suspended in a pharmaceutically acceptable vehicle for injection, suspensions ready for injection, and emulsions. In addition, controlled-release parenteral dosage forms can be prepared for administration to a patient, including, but not limited to, extended release tablets, pills or capsules, DUROS®-type and other implantable dosage forms, for systemic or tissue-specific delivery. Suitable vehicles that can be used to provide parenteral dosage forms include, without limitation: sterile water; water for injection USP; saline solution; glucose solution; aqueous vehicles; water-miscible vehicles; and non-aqueous vehicles. Compounds that alter or modify the solubility of a pharmaceutically acceptable salt of an active can also be incorporated into the parenteral dosage forms, including conventional and controlled-release parenteral dosage forms. Further additional agents may contain, e.g. anti-oxidants, buffers, bacteriostats, and solutes, which render the formulations isotonic with the blood of the intended recipient. The formulations may include aqueous and non-aqueous sterile suspensions, which contain suspending agents and thickening agents. Sterile injectable preparations, for example, injectable aqueous or oleaginous suspensions, can be formulated as is well known in the art, e.g. using suitable dispersing or wetting agents and suspending agents.

In embodiments, dosage forms suitable for oral or sublingual administration include tablets, troches, capsules, elixirs, suspensions, syrups, wafers, chewing gum, or the like, prepared as is well known in the art. The amount of active in such dosage forms may be adjusted as will be apparent to a person of ordinary skill, e.g. depending on the frequency of administration desired, and whether an extended release formulation is prepared. A syrup formulation will generally consist of a suspension or solution of the active or its salt in a liquid carrier, for example, ethanol, glycerine or water, with a flavoring or coloring agent.

In embodiments, solid dosage forms for oral administration include, e.g., capsules, tablets, pills, powders, and granules. In such solid dosage forms, the active is mixed with at least one inert a) filler, extender or diluent (e.g. starch, lactose, sucrose, glucose, mannitol, silicic acid, and mixtures thereof), and b) binder (e.g. carboxymethylcellulose, alginate, gelatin, polyvinylpyrrolidinone, sucrose, acacia, and mixtures thereof), c) humectant (e.g. glycerol), d) disintegrating agent (e.g. agar-agar, calcium carbonate, potato starch, tapioca starch, alginic acid, certain silicates, sodium carbonate, and mixtures thereof), e) solution retarding agents (e.g. paraffin), f) absorption accelerators (e.g. quaternary ammonium compounds, and mixtures thereof), g) wetting agents (e.g. cetyl alcohol, glycerol monosterate , and mixtures thereof), and h) absorbents (e.g. kaolin clay, bentonite clay, and mixtures thereof), and i) lubricants (e.g. talc, calcium stearate, magnesium stearate, solid polyethylene glycols, sodium lauryl sulfate, and mixtures thereof), and mixtures thereof. Such dosage forms can also include additional substances, e.g., tableting lubricants and other tableting aids such as magnesium stearate and microcrystalline cellulose, or buffering agents, in particular e.g. in capsules, tablets and pills. Solid compositions of a similar type can also be employed as fillers in soft and hardfilled gelatin capsules using excipients such as, e.g., lactose or milk sugar as well as high molecular weight polyethylene glycols. Alternatively or additionally, the actives can be in micro-encapsulated form with one or more excipients as noted above.

The various solid dosage forms (e.g. tablets, dragees, capsules, pills, and granules) can be prepared with coatings and shells such as enteric coatings, release controlling coatings and other coatings well known in the pharmaceutical formulating art. They can optionally contain opacifying agents and can also be of a composition that they release the active only, or preferentially, in a certain part of the intestinal tract, optionally, in a delayed manner. Examples of embedding compositions that can be used e.g. for delayed or extended release include polymeric substances and waxes.

In embodiments, the actives and in particular the one or more THAD can be present in form of salts, which may be particularly suitable for use in the treatment of cancer. The salts of the present invention may be administered to the patient in a variety of forms, depending on the route of administration, the salt involved, and the cancer being treated. For example, an aqueous composition or suspension of the salts may be administered systemically or tissue-specifically by injection, e.g. in the form of a pharmaceutical matrix by injection or surgical implantation, at a desired site. The particular technique employed for administering the matrix may depend, for example, on the shape and dimensions of the involved matrix. In some embodiments, the salt is introduced substantially homogeneously in a tumor to minimize the occurrence in the tumor of cold (untreated) areas. In certain embodiments, the salt is administered in combination with a pharmaceutically acceptable carrier. A wide variety of pharmaceutically acceptable carriers are available and can be combined with the salts, as will be apparent to one of ordinary skill in the art.

In embodiments, effective amounts, toxicity, and therapeutic efficacy of the active and/or its dosage form can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dosage can vary depending upon the dosage form employed and the route of administration utilized. The dose ratio between toxic and therapeutic effects is the therapeutic index and can be expressed as the ratio LD₅₀/ED₅₀. A therapeutically effective dose can be estimated initially from cell culture assays. Also, a dose can be formulated in animal models to achieve a circulating plasma concentration range that includes the IC₅₀ (i.e., the concentration of the compound of the invention, which achieves a half-maximal inhibition of symptoms, in case of cancer e.g. inhibition of the growth of the cancer cells) as determined in cell culture, or in an appropriate animal model, in particular mammalian animals, including e.g. mouse, rat, guinea pig, rabbit, pig, dog, and others. Levels in plasma can be measured e.g. by high performance liquid chromatography (HPLC). The effects of any particular dosage can be monitored by a suitable bioassay well known in the pharmaceutical art.

In embodiments, the dosage of a pharmaceutical formulation as described herein can be determined by a physician and adjusted, as necessary, to suit observed effects of the treatment. With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment, or make other alterations to the treatment regimen. The dosing schedule/regimen can vary, e.g. once a week, daily, or in particular predetermined intervals, depending on a number of clinical factors, including e.g. the subject's sensitivity to each of the actives.

In embodiments, a pharmaceutical composition comprising one or more active (i.e. one or more THAD, optionally combined with one or more further/secondary drug, in particular cancer drug) can be administered to a patient, or to the patient's tumor, cancer or pre-cancerous cells, either in vivo or in vitro, in an effective dose, in particular, to inhibit or suppress cancer cell growth, or optionally induce apoptosis, and thus treat the cancer or tumor, or to lower the risk of cancer developing or a tumor increasing its growth, e.g. in particular groups of patients at higher than average risk for cancer and/or tumor development. The one or more active may be concurrently administered, or may be administered according to a particular dosing regimen, e.g. as described herein-above. The dosing regimen typically takes into account factors such as the concentration of the active(s) in the blood and the half-life of each active, as will be apparent to a person of ordinary skill.

In embodiments, an effective dose of a pharmaceutical composition comprising the one or more active can be administered to a patient once or repeatedly. The pharmaceutical composition can also be administered over a period of time, such as over a 5 minute, 10 minute, 15 minute, 20 minute, or 25 minute period. If warranted, the administration can be repeated, for example, on a regular basis, such as hourly for 3 hours, 6 hours, 12 hours or longer or such as biweekly (i.e., every two weeks) for one month, two months, three months, four months or longer. In some instances, after an initial treatment regimen, the subsequent treatments can be administered on a less frequent basis. For example, after administration biweekly for three months, administration can be repeated once per month, for six months or a year or longer. Administration of a composition comprising one or more active in a coordinated administration schedule may be adjusted accordingly to ensure exposure to a plurality of actives, e.g. one or more active and/or a secondary drug, in particular a cancer drug), e.g. so that a reduction of levels of a biomarker or one or more symptom results, e.g. anti-cancer effects such as growth inhibition of cancer or tumor cells, growth inhibition of a tumor, reduction of tumor volume/tumor shrinkage, reduction of metastase formation, or reduction of the risk thereof.

In embodiments, the amount and/or concentration of the one or more active may depend on the typical dosage of the particular formulation and its route of administration, and may be adapted to expose the tumor, cancer or pre-cancerous cells to concentrations of, e.g., from about 0.1 to about 100 µM. For example, for the one or more THAD, the concentration may be about 0.5 to about 50 µM, e.g. about 16 µM or about 32 µM. All amounts and concentrations will need adaptation to factors including the circumstances of the individual patient, cancer type, and treatment duration, as will be apparent to a person of ordinary skill.

In embodiments, the amount and/or concentration of the one or more active in the pharmaceutical composition can be based on weight, moles, or volume. In embodiments, the pharmaceutical composition may comprise about 0.01%-99%, 0.05%-90%, 0.1%-85%, 0.5%-80%, 1%-75%, 2%-70%, or 3%-65%, 4%-60%, or 5%-50% of the one or more THAD and/or further active. The pharmaceutical composition may comprise at least 0.0001%, at least 0.1%, 0.5%, 1%, 2%, 3%, 4%, 5%, 10%, or at least 15% of each THAD and/or active. Alternatively or additionally, the pharmaceutical composition may comprise a maximum of up to about 0.0001%, 0.1%, 0.5%, 1%, 2%, 3%, 4%, 5%, 10%, or 15% of each THAD and/or active.

In embodiments, provided is a bifunctional method of cancer therapy or cell inhibition (inhibiting the growth or development of cancerous or pre-cancerous cells, shrinkage of tumors) and identification/localization, e.g. by imaging, in particular NIR imaging. Therein, the cancerous cells, pre-cancerous cells, or tumors are additionally identified, imaged and/or localized in a patient in need of such therapy. The method may comprise providing one or more THAD (and optionally further active or cancer drug) and administering it to a patient, and performing optical imaging for the THAD. The method may comprise providing one or more THAD and administering it to a patient; and performing optical imaging for the THAD. This allows to visually follow the progress of cell growth inhibition or treatment (e.g. growth arrest, disappearance or shrinkage of tumors, cancer cells, or pre-cancerous lesions), which in turn allows to adjust dosage of the one or more THAD and optional secondary actives, and/or to determine the location of tumor(s) and/or metastase(s) within the NIR spectral region of the THAD. In various embodiments, imaging may be performed, for example, about 6 to 48 hours post administration, e.g., without limitation, by injection. Imaging may be performed by comparing NIR signals of cancer/tumor cells to a background signal determined by imaging normal tissue/cells.

In embodiments, provided is a bifunctional method of conducting in situ pharmacokinetic and pharmacodynamic analyses of the THAD and/or further actives in a tumor, cancer or tumor cells, or normal cells or tissue. The method can comprise providing the one or more THAD (and optionally further actives or cancer drugs); contacting it with the cancer/tumor cells or tumor, or with normal cells or normal tissue; and subsequently imaging the cells exposed to the THAD, followed by pharmacokinetic and/or pharmacodynamics analyses, e.g. determining the fluorescence (or changes thereof) over time.

In embodiments, provided is a method wherein one or more one or more loading compound and one or more maintenance compound are co-administered to the patient in a coordinated administration schedule of one or more combined dosage forms, wherein each dosage form comprises one or more loading compound and one or more maintenance compound, and one or more pharmaceutical excipient. The maintenance compound may be one or more HMCD-DHA-mono-ester, which may be combined with a THAD loading compound selected from a HMCD-DHA-mono-ether, HMCD-DHA-bis-ether, HMCD-DHA-bis-ester, HMCD-DHA-bis-carbamate or HMCD-DHA-bis-thiocarbamate, as explained in more detail for illustrative co-administration schedules further below. Alternatively, the maintenance and loading dosages may be provided separately and sequentially in a coordinated dosing schedule, with the loading dosage preceding the maintenance dosage by 1-5 hours.

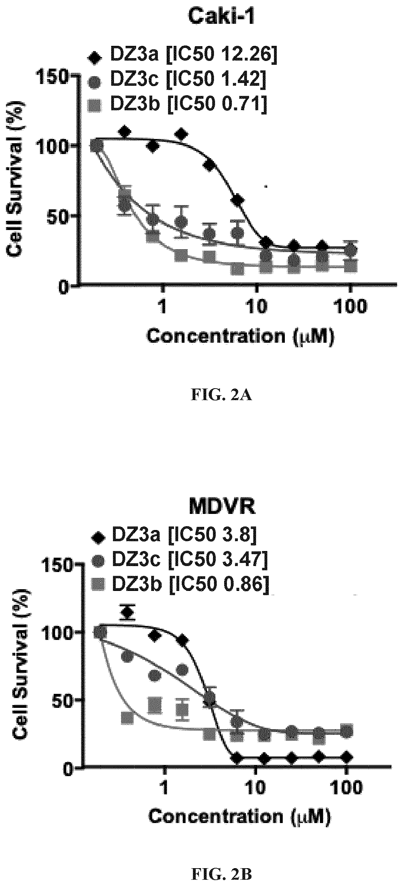

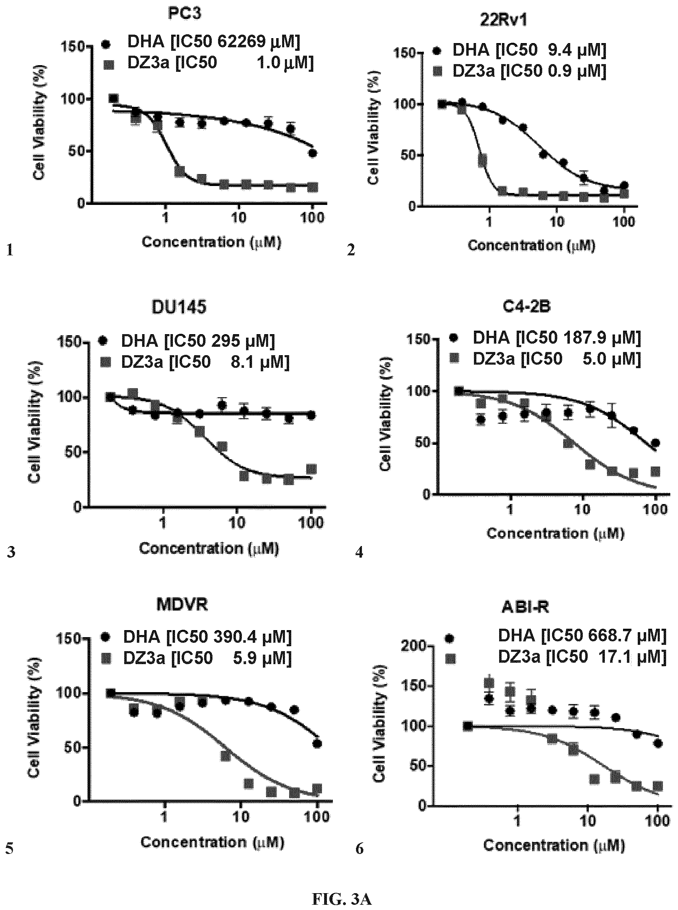

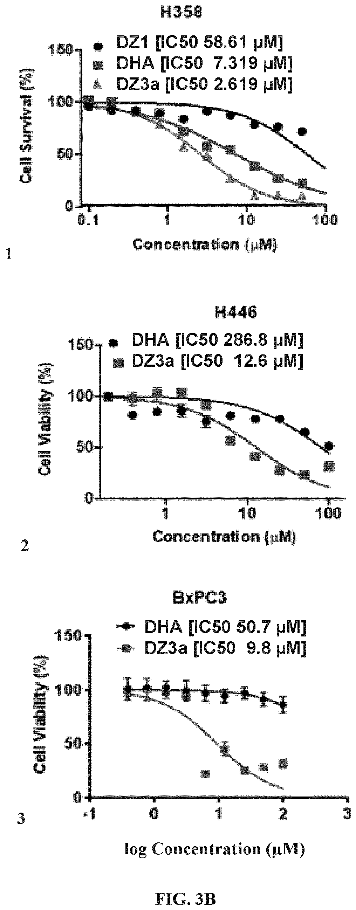

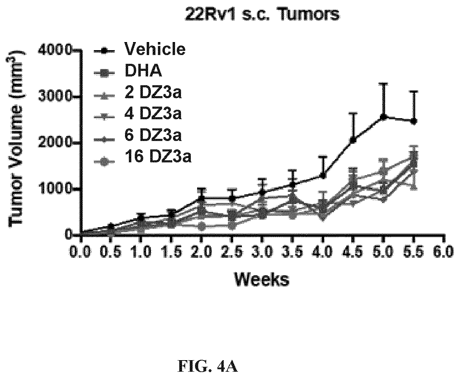

Improvements of the THAD are illustrated in **FIG. 2A** and **FIG. 2B** that show that THAD can provide improved cancer cell growth inhibition compared to a DHA-mono-ester; growth inhibition and *in vivo* tumor shrinkage of the DHA-mono-ester compared to both the unconjugated DHA itself, and compared to the unconjugated dye are shown in **FIG. 3A** and **FIG. 3B****,** and *in vivo* in **FIG. 4A**); the effects are shown in different cancer cell lines and human tumors grown in mice. Also as illustrated in **FIG. 2B****,** THAD can provide improvements in treating drug-resistant cancer cells.

Surprisingly, the THAD seems to provide a sigmoidal dose-response curve that is less steep compared to other conjugates such as the DHA-mono-ester, as indicated by preliminary data and e.g. in **FIG. 2A and FIG. 2B****.** This means for the range of doses which provide an initial effect, the THAD provides this initial effect at a lower dosage, at which e.g. the mono-ester does not have any effect yet (but may cause side effects).

Without wishing to be bound by theory, it is believed that THAD may have a longer tumor residence time compared to the DHA-mono-ester. The increased tumor residence time together with a less steep sigmoidal dose-response curve (and thus lower dose at which an initial non-maximal effect occurs) may provide a sufficient therapeutic effect for the THAD.

Without wishing to be bound by theory, it is believed that THAD may have an improved dose-response curve compared to the DHA-mono-ester that is less steep, i.e. providing an initial effect at a lower dose and/or more quickly as indicated by preliminary data and e.g. in **FIG. 2A and FIG. 2B****.** Without wishing to be bound by theory, it is believed that in THAD an early systemic release may be avoided yet the anti-cancer effects may be provided immediately within the first few hours, as these effects are mediated by a relatively stable, or alternatively slow releasing conjugate, wherein any substantial amount of the released DHA portion would occur substantially non-systemically after cancer cell entry of a THAD, in particular, an ether-conjugated, carbamate conjugated and/or thiocarbamate conjugated THAD, including mono-DHA and bis-DHA ether, carbamate and/or thiocarbamate conjugates.

Further, without wishing to be bound by theory, it is believed that THAD are able to effectively treat the most aggressive cancers that tend to develop resistance and other resistance-prone forms of cancer, and are able to do so without requiring co-administration of cancer drugs such as TKI or chemotherapeutics; thus THAD may be effective in cancers such as, without limitation, prostate cancer, kidney cancer, pancreatic cancer, lung cancer including non-small cell lung cancer (NSCLC), adenocarcinomas (AC) of the lung, and small cell lung cancer (SCLC). This is shown directly for THAD e.g. in **FIG. 2A, FIG. 2B****,** and by way of the DHA-mono-ester, which, without wishing to be bound by theory, is believed to be outperformed by THAD based on preliminary data from a variety of cancer cells lines; the lesser DHA-mono-ester effects are shown e.g. in **FIG. 3A****,** **FIG. 3B****,** and **FIG. 4A****.** Thus it is believed superior growth inhibition using THAD may be achieved in a wide variety of human cells lines not only including kidney cancer, clear cell renal cell carcinoma cells, prostate cancer, prostate carcinoma, and Enzulamide-resistant prostate cancer, but also prostatic adenocarcinoma, lung cancer, lung carcinoma, NSCLC, lung adenocarcinoma, SCLC, pancreatic cancer, pancreatic adenocarcinoma, and including various drug resistant forms, and in mouse models for the human tumors formed in mice implanted with these human cancer cells. Without wishing to be bound by theory, THAD (including one or more of bis-ether-derivatives, bis-ester derivatives, bis-carbamate derivatives, bis-thiocarbamate derivatives, mixed derivatives, or combinations thereof) are believed to outperform the DHA-mono-ester in some or all of these cancer types, in particular in their dose-response. Treatment of these cancer forms with THAD thus may allow to decrease side effects and/or the risk of development of resistance that is often encountered with standard treatments such as TKI or chemotherapeutics, or to overcomes resistance once developed, e.g. resistance due to prior cancer drug treatment, for example with tyrosine kinase inhibitors (TKI) such as Gefitinib or standard chemotherapeutics such as Docetaxel and Cisplatin (DDP).

Furthermore, without wishing to be bound by theory, based on preliminary data, it is believed that the THAD cross the blood-brain-barrier and are thus able to treat brain tumors and brain metastases even when administered systemically, rather than requiring local administration.

Without wishing to be bound by theory, it appears that THAD, when their effect such as growth inhibition is compared to the respective DHA-mono-ester, have a dose response curve that is less steep and start to be effective at a lower concentration and provide an initial non-maximal effect. For example, the dose of the THAD may be, e.g., about 0.1 mg to a maximum about 6 mg/kg or less (e.g. up to about 2.5 mg or even about 1.0 mg/kg).

In some cancers such as kidney cancer, the maximal effect achieved outperforms that of the DHA-monoester, compared e.g. **FIG. 2A****,** in which case one or more THAD may be administered for treatment.

Alternatively, in some cancers, while the dose-response curve of the THAD is improved the maximal anti-cancer effect may be lower than that of the DHA-mono-ester. Due to its long tumor residence time the THAD may continue to have a non-maximal but sufficient therapeutic effect. Alternatively, the THAD may be used in combination with one or more DHA-mono-ester to thus provide a sufficient therapeutic effect earlier in time at a given total dose, lowering the total dose of active at which a sufficient or maximum effect is achieved, and/or increasing the effect achieved with a given dose. Illustrative co-administration schedules of THAD and DHA-mono-ester are described herein-below.

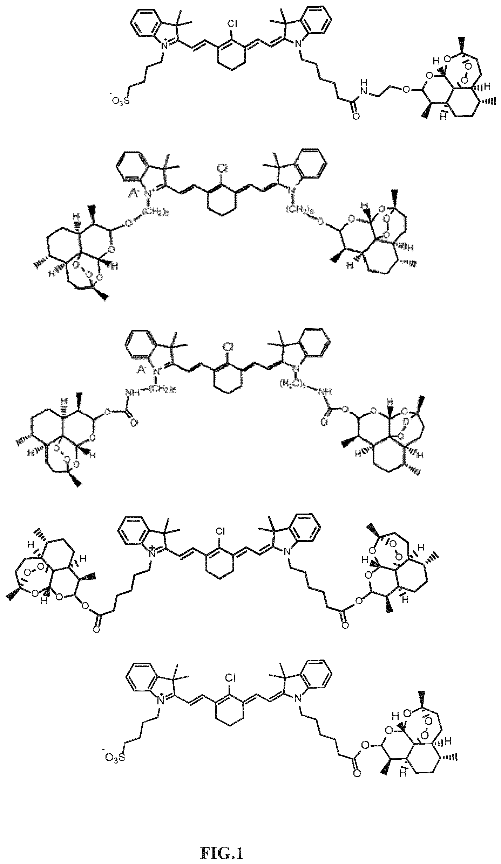

The DHA-monoester is a compound having the structure of formula FI wherein the ether bond linking the HMCD dye residue to the DHA is replaced with an ester bond, for example, without limitation, as shown for DZ3a (see e.g. compound 3 in **FIG. 1** for a HMCD-DHA monoester wherein X is Cl, R₁ and R₂ each is H, and wherein R₃ is a - (CH₂)4-SO₃⁻ alkylsulphonate residue.

Without wishing to be bound by theory, it is believed that THAD may have a longer tumor residence time compared to the DHA-mono-ester. The increased tumor residence time together with a less steep sigmoidal dose-response curve (and thus lower dose at which an initial non-maximal effect occurs) may provide a sufficient therapeutic effect for the THAD. Thus the one or more THAD may be administered either alone, or may be administered together with a dose of DHA-mono-ester that is lower than the dose that provides the maximal effect when administered on its own, or may be co-administered as described herein, e.g. as one or more subsequent maintenance dose following one or more initial THAD loading dose.

In addition, without wishing to be bound by theory, specific advantages of the THAD may include their substantial *in vivo* stability or alternatively a comparatively slow continuous and thus mostly non-systemic DHA release in cancer cells and/or tumor tissue that allows systemic administration in form of the THAD while avoiding or reducing DHA side effects associated with systemic administration of an unconjugated DHA. Without wishing to be bound by theory, this may provide additional or improved anti-cancer effects not provided by HMCD-DHA-monoester conjugate, in particular DZ3a. Again without wishing to be bound by theory, additional or improved anti-cancer effects may be provided by co-administering one or more of a THAD selected from mono-ether, bis-ether, bis-ester and bis-carbamate/bis-thiocarbamate bis-DHA with a HMCD-DHA-monoester. For example, a mono-ether or bis-ester may be co-administered with a mono-ester. In the co-administration, a maintenance compound (MC), in particular, the mono-ester, may serve as maintenance doses that accompanies or follows an initial loading dose, as described herein below.

For example, the advantages and improvements of the THAD may allow for an improved administration schedule with less frequent instances of drug administration, including a co-administration schedule wherein a THAD and a HMCD-DHA-monoester are combined. For example, without limitation, the THAD may be administered first, providing a loading dose, which is followed by one or more subsequent maintenance doses of an ALSD.

Without wishing to be bound by theory, the THAD may provide a sigmoidal dose-response curve that is much less steep compared to the DHA-mono-ester, as indicated by preliminary data and e.g. in **FIG. 2A,** and **FIG. 2B****.** This means for the range of doses which provide an initial effect, the THAD provides this initial effect at a lower dosage, at which the HMCD-DHA-monoester does not have any effect yet (but may cause side effects). Similarly, looking at the range of higher doses that provide maximum effect, the monoester provides the maximum effect at a lower dose while more of the THAD may be needed. Thus administration of the THAD alone or in combination with a monoester, in particular according to the dosing and administration schedules described herein, may provide an improved therapy that provides increased cancer growth inhibition and/or reduced side effects, and allows to use a reduced dose of more toxic drug alternatives, including the HMCD-DHA-monoester, and/or less frequent drug doses e.g. co-administering THAD and HMCD-DHA-monoester in a particular ratio, amount and frequency adjusted to capture both the initial effect of the THAD and the maximum effect of the HMCD-DHA-monoester, in particular for types of cancer wherein the THAD may not provide a maximum effect (see **FIG. 2B**).

In embodiments, pharmaceutical kits are provided. Such kits can comprise one or more THAD or composition comprising a THAD, preferably in form of its salt, and, typically, a pharmaceutically acceptable carrier. The kit can also further comprise conventional kit components, such as needles for use in injecting the composition(s), one or more vials for mixing the composition components, and the like, as are apparent to those of ordinary skill in the art. In addition, instructions, e.g. as inserts or as labels, indicating quantities of the components, guidelines for mixing the components, and protocols for administration/co-administration, can be included in the kit. In particular, the kit may comprise instructions for a co-administration schedule of a plurality of THAD in a coordinated administration schedule as described herein below, including, without limitation, loading and maintenance dosage details such as amounts and timing, and optionally, a HMCD-DHA-monoester.

In embodiments, the one or more THAD and optional one or more maintenance compound (MC), i.e. one or more HMCD-DHA-monoester, may be co-administered in a coordinated administration schedule either concurrently or subsequently. For example, a loading dose of the THAD may be administered first in a first time interval, followed by one or more subsequent maintenance dose(s) of a second THAD or a HMCD-DHA-monoester at the start of a second time interval. For example, a first amount of about 0.1 mg to about 10 mg THAD/kg body weight, preferably about 0.1 to about 6 mg/kg, e.g. about 0.1 to about 2.5 mg or about 0.1 mg to about 1.0 mg/kg may be administered to a patient, depending on the route of administration, e.g. either intravenously or orally or by any convenient administration means (loading dose). The THAD loading dose may be separated into multiple doses during the first time interval, for example, 4 to 72 hours, e.g. about 4 h, 8 h, 16 h, 24 h, 32 h, 48 h, or 72 h. Advantageously, the loading dose of the THAD is lower than the maintenance dose of the 2^{nd} THAD or HMCD-DHA-monoester. Without wishing to be bound by theory, it is believed that the THAD is effective at a lower concentration compared to the HMCD-DHA-monoester, and allows to prime to body to experience the THAD effects, while the HMCD-DHA-monoester, especially when used on its own, may require a higher dose (but at a higher dose may provide a better maximum effect compared to the THAD).

In embodiments, during a subsequent second and optional further subsequent time intervals, one or more maintenance doses of a maintenance compound (e.g. a HMCD-DHA-monoester or a second THAD, e.g., without limitation, the bis-ester) may be administered. For example, the one or more maintenance dose may be administered in an amount, per dose, of about 0.1 mg to about 10 mg/kg body weight, preferably about 1 to about 10 mg/kg, e.g. about 1 to about 8 mg, about 1 to about 6 mg, about 1 mg to about 4 mg, or about 1 to about 2 mg/kg may be administered to a patient, depending on the route of administration and frequency of administration. The subsequent dose(s) may be a single subsequent dose, or multiple subsequent doses at the same or different intervals, e.g. bi-daily, daily, over 2-7 days, weekly, etc. Preferably, the THAD loading dose may be administered on day 1, and after about 24 hours on day 2, one or more higher maintenance dose(s) may be administered as described herein, e.g. in daily or in less frequent time intervals. Each maintenance dose may be higher or lower compared to the loading dose of the THAD. Suitable ratios of THAD:maintenance compound (MC) may include, for example, from about 20:1 to about 1:20 (THAD:MC), for example about 10:1 to about 1:10 (THAD:MC), e.g. about 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or about 10:1 (THAD:MC). Advantageously, the loading dose of the THAD is lower than the maintenance dose of the MC.

Alternatively, the THAD loading dose may be co-administered concurrently with an maintenance dose of a maintenance compound within a first time interval, and at the start of the second time interval may be followed by one or more maintenance doses as described above.

Still alternatively, the THAD and the maintenance compound may be co-administered in a particular ratio, either for each dose (including the first dose in the first time interval), or for the maintenance dose(s) starting at the second time interval only. This ratio may be from about 20:1 to about 1:20 (THAD:MC), for example about 10:1 to about 1:10 (THAD:MC), e.g. about 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or about 10:1 (THAD:MC).

In embodiments, the THAD (or a combination of an THAD with an MC or 2^{nd} THAD, co-administered as described above), may be co-administered with further drugs in a co-administered in a coordinated administration schedule either concurrently or subsequently, as described herein-above.

Without wishing to be bound by theory, it is believed that the HMCD moiety and the DHA moiety when linked as described are able to act in concert to achieve effects that are at least additive and possibly synergistic, and may provide or contribute to the effects of DHA derivatives, such as the DHA mono-ester, and particularly to the improved therapeutic effects of the THAD. This may be due to one or more of the following three types of actions or functions of the HMCD-DHA conjugates, but not the unconjugated HMCD dye: 1) mitochondrial functions, 2) lysosomal functions, and 3) cell-cell communication through protein prenylation. These three functions or actions occur independently, but collectively contribute to multiple mechanisms of THAD each of which contributes to improved inhibition of the growth of cancer cells.

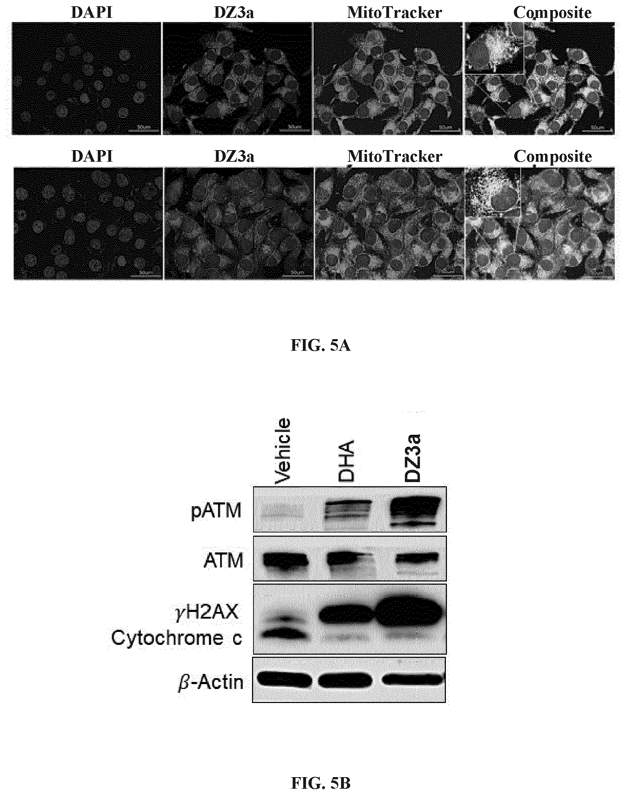

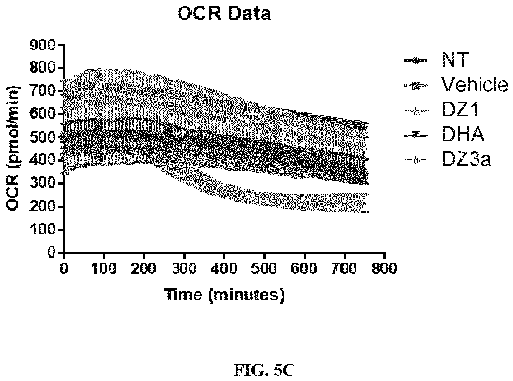

The three functions of the HMCD-DHA conjugates (unlike the unconjugated HMCD dye or the unconjugated DHA) are believed to display are supported by the results shown in **FIG. 5A-G****.** With regard to 1) and 2) a staining of cancer cells shows that HMCD-DHA conjugates co-localize with mitochondria and lysosomes (see **FIG. 5A**), and thus are able to interfere with various mitochondrial and lysosomal functions in cancer cells. Western blot analysis shows that HMCD-DHA conjugates can induce DNA damage, and deplete mitochondria (see **FIG. 5B**). **FIG. 5C** show an incredible improvement in lowering the oxygen consumption rate (OCR) of cancer cells when compared to the unconjugated dye or DHA, or controls. Also with regard to 1) and corresponding with a strongly decreased OCR, the THAD also show a decrease of the extracellular acidification rate ("ECAR") (data not shown). The ECAR corresponds to the use of anaerobic glycolysis, which in its anaerobic form produces and accumulates lactic acid extracellularly, thus the higher rate of extracellular acidity build-up by lactic acid. While all samples show a decrease in ECAR (anaerobic ATP production), the highest decrease is exhibited by the HMCD-DHA conjugates. A lowering of the ECAR typically indicates less anaerobic glycolysis (and more aerobic respiration instead). The anaerobic production of ATP, i.e. via the anaerobic glycolysis/lactic acid system, is an alternative means of cell survival that is particularly important for cancer cells and especially in solid tumors where oxygen may not be readily available. As determined, HMCD-DHA conjugates effects physiological changes in cancer cells which include a strong reduction of aerobic and also anaerobic ATP production, which contributes to inhibit the growth of cancer cells.

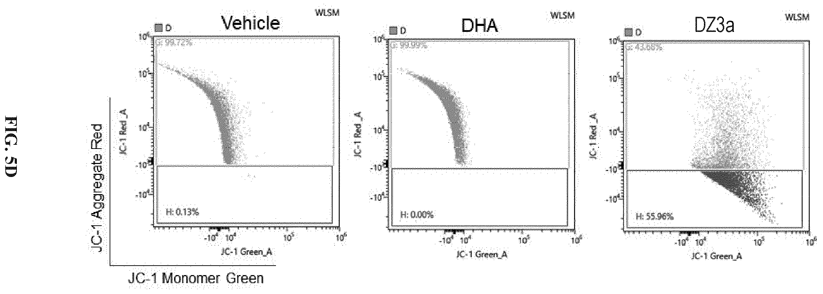

With regard to 1), it is shown that DHA conjugates can achieve a reduction in respiration due to cell membrane damage which allows protons to leak out (see **FIG. 5C**); further they can achieve an increased polarization of the membrane potential, and thus increased damage to mitochondria (see **FIG. 5D**).

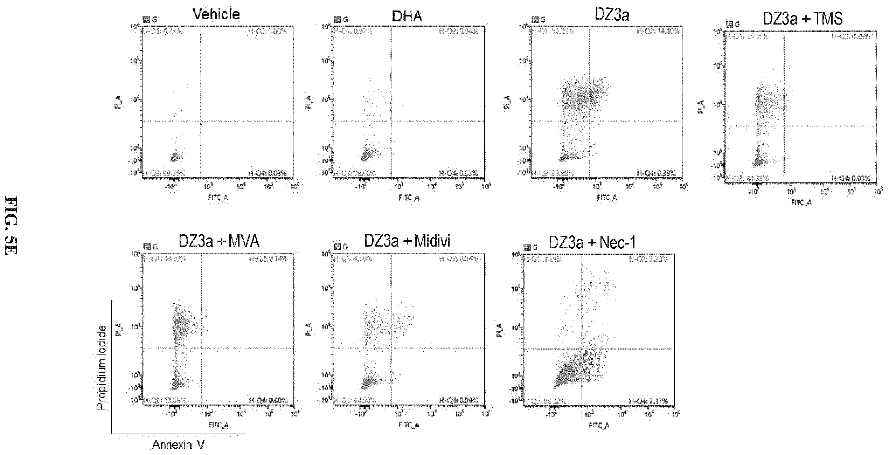

That DHA conjugates can achieve whole cell damage and cell death (including apoptosis) is shown in **FIG. 5E****;** in the lower left quadrant the blue signals designate healthy cells, green signals designate damaged cells/cell death (rather than apoptosis), and pink signals designate apoptosis; purple signals designate early apoptosis (some leakage but the cells are still alive and can recover).

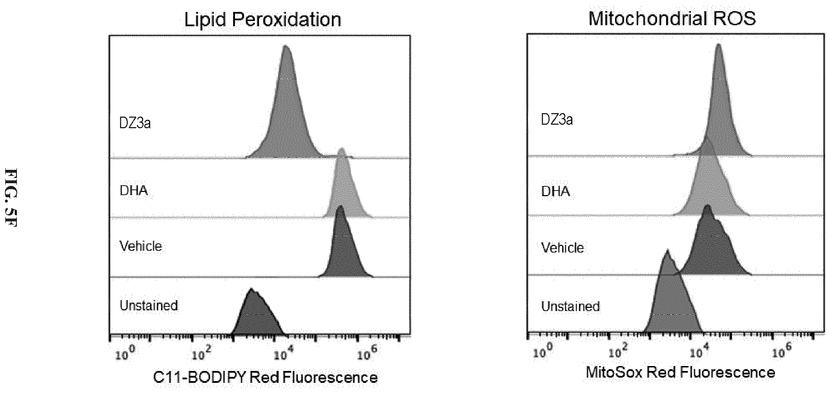

DHA conjugates can act via ferroptosis as is shown in **FIG. 5F****;** Ferroptosis is an iron-dependent oxidative form of cell death associated with increased lipid peroxidation and insufficient capacity to eliminate lipid peroxides, which may be contributed to by a loss of activity of the lipid repair enzyme glutathione peroxidase 4 (GPX4). In **FIG. 5F****,** mitochondrial ROS is shown by a red shift to the right, while a shift to the left is the reverse, i.e. increased lipid peroxidation; thus DHA appears to engage ferroptosis/iron-related mechanisms of cell death which are distinct from apoptosis.

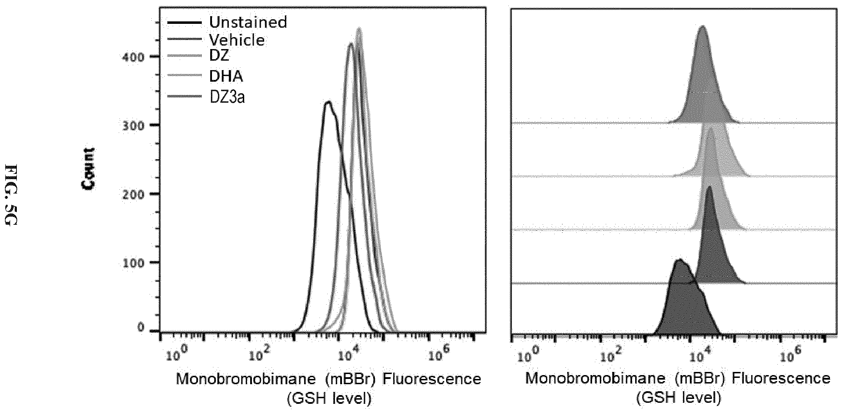

DHA conjugates can also reduce the cell's ability to produce antioxidants (e.g. glutathione (GSH); ROS reactive oxidant species lower the cell's defense mechanism against such reactive species thus further contributing to cell damage (see **FIG. 5G**).

Without wishing to be bound by theory, THAD thus may provide various advantages compared DHA in its unconjugated form, compared to unconjugated HMCD; further, improvements may be provided by the particular link of the conjugate, e.g. THAD may provide one or more improvements as described herein compared to the DHA-mono-ester. Such improvements may also include one or more of a reduced general cytotoxicity in normal cells combined with an increased cytotoxicity in cancer cells (as shown e.g. by the IC₅₀), increased anti-cancer effectiveness (e.g. growth inhibition, tumor shrinkage, more rapid growth inhibition/cell death of cancer cells, e.g. less than 16, 12, 10, or 8 hours, even when tested on drug-resistant cells, thus avoiding the development of drug resistance), shorter duration of administration, less frequent administration schedule (including e.g. just once, once-weekly, bi-weekly, monthly etc.), decreased side effects (particularly when administered systemically), reduced future risk (such as risk for cancer, metastasis and chemotherapeutical induced disease), decreased and/or slower drug inactivation (particularly when administered systemically), an improved plasma and/or elimination half-life (e.g. of less than 8, 4, 2, 1 hour or 30 minutes, e.g. about 1-2 hours), increased plasma circulation time, increased tumor residence time (e.g. longer than 1, 2, 3, 4 weeks, or longer), and an improved dose-response curve, in particular, a less steep sigmoidal dose response curve.

### DETAILED DESCRIPTION OF THE DRAWINGS

Turning to **FIG. 1****,** the chemical structures of the DZ1-DHA-ether, of the MHI-148-bis-DHA-ether, -carbamate and -ester, and for comparative purposes of the mono-ester conjugate (DZ1-DHA-ester), are shown.

In **FIG. 2A****,** improved dose response and growth inhibition of kidney cancer cells (here clear cell renal cell carcinoma cells, specifically Caki-1) is shown. The graphs illustrate IC₅₀ curves for treatment of the cancer cells with DZ1-DHA-ether (circles) or MHI-148-bis-DHA-ester (squares), both of which display a favorable very gradual dose-response curve and an IC₅₀ of 0.5-1.5, with MHI-148-bis-DHA-ester outperforming DZ1-DHA-ether. In comparison, the graph of the DZ1-DHA-mono-ester (diamonds) at above 12 shows a much higher IC₅₀ and a much steeper dose-response curve than both the mono-ether and the bis-ester, requiring a much higher concentration to start to show an effect. Even at double the concentration, the effects on cancer cell survival are less.

In **FIG. 2B****,** improved dose response of Enzalutamide (ENZ)-resistant prostate cancer cells (here ENZ-resistant C4-2B cells (MDVR)) is shown. The graphs illustrate IC₅₀ curves for treatment of the cancer cells with DZ1-DHA-ether (circles) or MHI-148-bis-DHA-ester (squares), both of which display a favorable more gradual dose-response curve compared to the mono-ester (diamonds). The IC₅₀ of the DZ1-DHA-ether is slightly improved (3.47 compared to 3.8 for the ester). MHI-148-bis-DHA-ester provides an even more gradual dose response and a much lower IC₅₀ of below 1, outperforming both the mono-ether and the mono-ester. In comparison, DZ1-DHA-ester has a much higher IC₅₀ at 3.8 and a much steeper dose-response curve, requiring a much higher concentration to start to show an effect.

In **FIG. 3A****,** panel 1-6 show growth inhibition of different prostate cancer cells (PC3, 22Rv1, DU145, C4-2B, MDVR, Abi-R) treated with DZ1-DHA-mono-ester compared to DHA.

In **FIG. 3B****,** panels 1-3 show growth inhibition of lung (H358, H446) and pancreatic cancer cells (BxPC3) treated with DZ1-DHA-mono-ester compared to DHA or compared to DZ1 and DHA/Artemisinin, respectively.

In **FIG. 4A****,** the graph in this comparative example shows tumor volume of human prostate tumors (22Rv1 cells) in nude mice, and volume increase over 5.5 weeks in the negative control (vehicle) and its inhibition by DZ-DHA-monoester in doses ranging from 2 to 16 mg/kg compared to 4.7 mg/kg DHA. The DHA-monoester conjugate performs similar to DHA, both similarly inhibit increase in tumor volume compared to the neg. control (vehicle). Surprisingly, the lowest dose of DZ-DHA, here about 2 mg/kg, shows better tumor inhibition than other higher doses of DZ-DHA.

In **FIG. 5A****,** it is shown that the DZ-DHA mono-ester (DZ3a) co-localizes with mitochondria and lysosomes and can thus interfere with various mitochondrial and lysosomal functions in cancer cells. The images of the fluorescent staining show the cell nuclei of human prostate cancer cells (MDVR, Enzalutamide-resistant C4-2B cancer cells) stained blue by DAPI, while the red fluorescence of the THAD co-localizes with that of green fluorescence of the MitoTracker™ and LysoTracker™ fluorescent dyes in the cytosolic fraction of the cancer cells, as shown by the yellow fluorescence in the composite. Preliminary data indicates that THAD similarly co-localize to mitochondria and lysosomes.

In **FIG. 5B****,** western blot analysis shows that DZ-003 induces DNA damage and depletes mitochondria as demonstrated by increased levels of pATM and γH2AX (DNA damage) and decreased levels of cytochrome c (mitochondrial marker).

In **FIG. 5C** it is shown that DZ3a can greatly lower the mitochondrial oxygen consumption rate ("OCR") of cancer cells compared to the unconjugated DHA, the unconjugated dye DZ1, or controls. The figure shows the OCR of C2-4B MDVR drug resistant cancer cells exposed to the following drugs: negative/blank control ("NT"), vehicle/DMSO control ("Veh"), the unconjugated dye ("DZ1"), the unconjugated DHA, and DZ3a. As illustrated, the rate drops down from about 700-800 pmol/min to less than 300 pmol/min for DZ3a, while the other drugs or controls drop much less. DZ3a thus significantly lowers the OCR starting its effect within about 250 minutes and continuing to lower further until about 500 minutes, then reaching a sustained lowered OCR; this significant lowering of the mitochondrial OCR is part of the effect on mitochondrial function exhibited, partly due to co-localization with these cell organelles.

In **FIG. 5D****,** the measured JC-1 fluorescence for its monomer and aggregate (on the x- and the y-axis, respectively), shows that DZ3a induces depolarization of mitochondrial membrane potential. This is in contrast to DHA, which has a similar effect as the negative control ("vehicle").

In **FIG. 5E****,** it is shown that DZ3a induces cell death which is blocked by mitochondrial fission (Midivi) and necroptosis inhibitors (Nec-1), and partially inhibited by an OATP inhibitor (TMA).

In **FIG. 5F****,** it is shown that DZ3a induces lipid peroxidation (as determined by decreased C11-BODIPY fluorescence (red), indicating lipid oxidation) and mitochondrial ROS (as determined by increased Mitosox fluorescence (red)), while DHA provides an effect similar to the control.

In **FIG. 5G****,** it is shown that DZ-003 decreases cellular antioxidant GSH levels.

In **FIG. 6A****,** an illustrative reaction scheme for synthesis for a DZ1-DHA-ether (DZ3c) is shown.

In **FIG. 6B****,** an illustrative reaction scheme for synthesis of a MHI148-bis-DHA-ester (DZ3b) is shown.

In **FIG**. **6C****,** an illustrative reaction scheme for synthesis of a DZ1a-bis-DHA-ether (DZ3d) is shown.

In **FIG. 6D****,** an illustrative reaction scheme for synthesis of a DZ1b-DHA-carbamate (DZ3e) is shown.

In **FIG. 6E****,** an illustrative reaction scheme for synthesis of a DZ1c-bis-DHA-carbamate (DZ3f) is shown.

In **FIG. 6F****,** an illustrative reaction scheme for synthesis of a DZ1b-DHA-thiocarbamate (DZ3g) is shown.

In **FIG**. **6G****,** an illustrative reaction scheme for synthesis of a DZ1c-bis-DHA-thiocarbamate (DZ3h) is shown.

### EXEMPLARY EMBODIMENTS

All chemicals and reagents may be purchased from standard sources such as Sigma-Aldrich. Deionized water (18.2 Ω) used for making solutions is obtained from Milli-Q Direct Ultrapure Water System from Millipore (Billerica, MA, USA). All intermediates are characterized by 1H NMR and mass analysis and the purity of compounds are analyzed by HPLC. 1H NMR data is collected on Bruker 400MHz spectrometers using standard parameters; chemical shifts are reported in ppm (δ) in reference to residual non-deuterated solvent. ESI mass spectroscopy analysis is performed on new compounds at Mass Spectrometry and Biomarker Discovery Core facility using a Thermo Fisher LTQ Orbitrap Elite system.

Cell culture: In the following examples, unless otherwise specified, all cell lines are purchased from American Type Culture Collection and cultured in American Type Culture Collection (ATCC)-recommended media, with fetal bovine serum (FBS) to a final concentration of 10 % and 1 × penicillin/streptomycin at 37°C with 5% CO₂ in a cell culture incubator, unless otherwise specified. Unless otherwise specified, culture is 2D. Where culture is 3D, low attachment plates are used, with the same media. **C4-2B** (ATCC® CRL-3315™, human prostate cancer cells of epithelial morphology) parental cell line and drug-resistant cells derived therefrom) are cultured in RPMI-1640 with 10% FBS. **MDVR cells** (an Enzalutamide-resistant variety of C4-2B prostate cancer cells formed as described below) are cultured as indicated for the parental C4-2B cells. **Caki-1** cells (human clear cell renal cell carcinoma cells) are cultured in ATCC-formulated McCoy's 5a Medium Modified (ATCC Catalog No. 30-2007) with 10% FBS. **PC3** cells (ATCC® CRL-1435™, a human prostate cancer cell line of epithelial morphology initiated from a bone metastasis of a grade IV prostatic adenocarcinoma) are cultured in F-12K with 10% FBS. **22RV1 Prostate Cancer (PC) cells** (**ATCC**® **CRL-2505™**, a human prostate carcinoma cell line of epithelial morphology) are cultured in RPMI-1640 with 10% FBS. **DU145** cells (ATCC® HTB-81™, a human cell line derived from a prostate-originating brain metastase, not detectably hormone sensitive, not expressing prostate antigen; forms adenocarcinoma grade II in nude mice) are cultured in ATCC-formulated Eagle's Minimum Essential Medium (EMEM, Catalog No. 30-2003). **H446** cells (ATCC® HTB-171™, a human small cell carcinoma SC lung cancer (SCLC) cell line derived from a metastatic site) are cultured in RPMI-1640 with 10% FBS. **H358** cells (ATCC® CRL-5807™, a human non-small cell lung cancer (NSCLC) cell line that expresses protein and RNA of SP-A, the major lung surfactant associated protein, and produces tumors in athymic nude mice) are cultured in RPMI-1640 Medium with 10% FBS. **BxPC3** cells (ATCC® CRL-1687™, an adenocarcinoma-derived pancreatic cancer cell line that expresses pancreas cancer specific antigen and carcinoembryonic antigen and forms tumors in nude mice) are cultured in RPMI-1640 with 10% FBS.

Resistant prostate cancer cell lines **MDVR** and **AbiR:** MDVR cells are Enzalutamide-resistant cells, AbiR are Abiraterone acetate resistant cells, formed from parental C4-2B prostate cancer cells as follows. A cell assay is performed exposing cells cultured as described above to various drugs indicated below for 72 hours. Parental C4-2B cells are C4-2B non-resistant cells not previously exposed to cancer drugs. Drug-resistant C4-2B cells are created by prolonged exposure to the relevant drug, i.e. Enzalutamide for MDVR, and Abiraterone acetate for AbiR cells, at initially sub-lethal and gradually increasing concentrations until resistance is achieved.
**Example 1:** A cell assay is performed exposing cells cultured as described above to various drugs indicated below for 24 hours unless otherwise indicated. Each of the cell lines is treated either with a THAD (e.g. DZ-DHA-ether, MHI-148-bis-DHA), or for comparison, with the DHA mono-ester "DZ-DHA"; alternatively/additionally, DZ1 and/or unconjugated DHA may serve as comparants. For each cell line, the IC₅₀ is determined for each drug in concentrations from 0 to 100 µM. Cell viability amd IC₅₀ is determined by an MTT assay as follows: 1× 10⁴/ml cells in 100 µl are treated with increasing concentrations of the drug or a control for 24 hours. In the controls (not shown in the figures), the cells are exposed to DMSO (vehicle) for a final concentration that equals the highest concentration of the drug tested, to a maximum concentration less than 0.1% v/v. 4 hours before culture end/SDS addition, 10 µL MTT (3-(4,5-Dimethyl-2-thiazolyl)-2,5-diphenyl-2H-tetrazolium bromide, Sigma-Aldrich) is added to wells containing the cells. At the end of culture, 100 µl 10% SDS is added and then the plate containing the cells is placed in a 37°C cell culture incubator for 8 hours. The absorbance density of the supernatant is read on a 96-well microplate reader at wavelength 595 nm. All IC₅₀ are relative IC₅₀, and based on the curves fitted as shown in the figures. The results of Caki-1 and MDRV are indicated in the table below along with preliminary results for further cancer cell lines (including, e.g., C4-2B, Abi-R, PC3, 22Rv1, DU145, H358, H446, BxPC3), and the corresponding curves are shown in **FIG. 2A** (Caki-1) and **FIG. 2B** (MDVR).

| Cell lines | **DZ-DHA-ether** | **MHI-148-bis-DHA** | **DZ-DHA (mono-ester)** |
|---|---|---|---|
| | IC₅₀ (µM) | IC₅₀ (µM) | IC₅₀ (µM) |
| **Caki-1** | 1.42 | 0.71 | 12.26 |
| **MDVR** | <3.47 | 0.86 | 3.8-5.9 |

**Examples 3A/B:** Cancer cell lines (PC3, 22Rv1, DU145, C4-2B, MDVR, AbiR, H358, H446, BxPC3) are treated with DHA and DZ-DHA (DZ-003) in concentrations up to 100 µM for 48h, and the IC₅₀ is determined as described in example 1. The IC₅₀ is indicated in the table below, and the corresponding IC₅₀ graphs are shown in **FIG. 3A** (prostate) and **FIG. 3B** (lung, pancreas). THAD as described herein can outperform the mono-ester DHA derivative (DZ-003), e.g. in dose response and/or growth inhibition and/or toxicity; this may also apply to cancer types such as prostate, lung (non-small cell lung cancer and small cell lung cancer), and pancreas; for example, without limitation, in the cell lines and corresponding cancer types listed in the table below.

| **Cell lines** | **DZ1-DHA (mono-ester)** IC₅₀ (µM) | **DHA** |
|---|---|---|
| ***Prostate*** | | |
| **PC3** | 1.0 | 62269.0 |
| **22Rv1** | 0.9 | 9.4 |
| **DU145** | 8.1 | 295.0 |
| **C4-2B** | 5.0 | 187.9 |
| **Abi-R** | 17.1 | 668.7 |
| **MDVR** | 5.9 | 390.4 |

| ***Lung*** | | |
|---|---|---|
| **H358** | 2.6 | 7.319 |
| **H446** | 12.6 | 286.8 |

| ***Pancreas*** | | |
|---|---|---|
| **BxPC3** | 9.8 | 50.7 |

**Example 4 A/B:** Human cancer cells are subcutaneously (s.c.) implanted (1 × 10⁶) into 4- to 6-week-old athymic nude mice (National Cancer Institute). When tumor sizes in the mice reach between 1 and 6 mm in diameter as assessed by *in vivo* bioluminescence imaging or by palpation, mice are injected once or multiple times i.p. with the drug, e.g. DZ3a, DHA or o THAD. For mice bearing 22Rv1 tumors, DZ3a (2, 4, 8 and 16 mg/kg) or DHA (e.g. 4.7 mg/kg) is injected twice a week for 5.5 weeks. Whole body optical imaging is taken at 24 hours or as indicated using a Kodak Imaging Station 4000 MM equipped with fluorescent filter sets (excitation/emission, 800:850 nm), with a field of view of 120 mm in diameter, a frequency rate for NIR excitation light of 2 mW/cm², and the following camera settings: maximal gain, 2 × 2 binning, 1,024 × 1,024 pixel resolution, exposure time of 5 seconds. Live mice are alternatively/additionally imaged by an Olympus OV100 Whole Mouse Imaging System (excitation, 762nm; emission, 800 nm; Olympus Corp.), containing a MT-20 light source (Olympus Biosystems) and DP70 CCD camera (Olympus). Before imaging, mice are anesthetized with isoflurane (2.5 units), and maintained in an anesthetized state during imaging. The results for DZ3a compared to DHA are shown in **FIG. 4A****.** Alternatively, a THAD may be compared to DZ3a. Preliminary experiments indicate that a THAD as described herein may inhibit prostate 22Rv1 subcutaneous tumors more efficiently compared to the mono-ester DHA derivative (DZ3a).
**Example 5A:** Confocal microscopy of Mitotracker/Lysotracker staining in cancer cells including drug-resistant C4-2B MDVR cells exposed to DZ3a is performed and shows exclusive targeting to mitochondria and lysosomes of the cancer cells (see **FIG. 5A**). Preliminary experiments indicated that THAD as described herein may similarly provide exclusive targeting to mitochondria and lysosomes. Subcellular localization (mitochondria, lysosomes) may be performed as follows: Uptake of DZ3a, THAD, controls or other dyes into mitochondria and lysosomes of cancer cells is determined by virtue of each compound constituting a dye or comprising a dye moiety. Cells are plated on live-cell imaging chambers (World Precision Instrument) overnight. Cells are exposed to the dyes at different concentrations, and dye uptake is evaluated by a Perkin-Elmer Ultraview ERS spinning disc confocal microscope mounted on a Zeiss Axiovert 200 m inverted microscope equipped with a 37°C stage warmer, incubator, and constant CO₂ perfusion. A 60× or 100× Zeiss oil objective (numerical aperture, 1.4) is used for live cell images, and a Z-stack is created using the attached piezoelectric z-stepper motor. The 633-nm laser line of an argon ion laser (set at 60% power) is used to excite the dye. Light emission at 650 nm is detected and found to correlate directly with the dye concentrations in the cells. For comparative studies, the exposure time and laser intensity are kept identical for accurate intensity measurements. Pixel intensity is quantified using Metamorph 6.1 (Universal Imaging), and the mean pixel intensity is generated as gray level using the Region Statistics feature on the software. To determine the dye uptake by mitochondria, the mitochondrial tracking dye MitoTrackerTM Green FM (InvitrogenTM Molecular ProbesTM) is used. To determine dye localization in lysosomes, lysosome-tracking dye LysoTrackerTM Green DND-26 (InvitrogenTM Molecular ProbesTM) are used. Imaging of mitochondrial and/or lysosome localization of the dyes (THAD, HMCD) is conducted under confocal microscopy.
**Example 5B:** Western blot analysis is performed to measure DNA damage, depletion of mitochondria, as demonstrated by increased levels of pATM and *γ*H2AX (DNA damage) and decreased levels of cytochrome c (mitochondrial marker). The western blot analysis is performed as follows: Protein lysates (25ug/sample) are separated by electrophoresis into the SDS-PAGE, which is then transferred to a nitrocellulose membrane for immunoblotting analysis. The protein membranes are blocked with 5% non-fat milk in PBS at room temperature (RT) for 1h, followed by incubation with primary antibodies against proteins of interests diluted in 2% BSA in Phosphate Buffered Saline with Tween-20 (PBST) at 1:500 or 1:1000 at 4°C overnight. The membranes are washed with PBST three times at 5 min for each wash on an orbital shaker, and then incubated with secondary antibodies against the species of the primary antibodies (i.e. anti-mouse or anti-rabbit) diluted in 5% non-fat milk in PBST for 1h at RT with shaking. After the secondary antibody, the membranes are washed three times for 5 min each, and subjected to chemiluminescence imaging with ECL substrates (e.g. Pierce ECL Plus™, Thermo Fisher Scientific), to detect the protein bands of interest. The results for DZ3a are shown in **FIG. 5B****.** Preliminary experiments indicate that the THAD as described herein may have an increased effect compared to DZ3a.
**Example 5 C:** To determine mitochondrial function of cancer cells (here C4-2B MDVR), the cells are treated with vehicle, DZ (5 uM), DHA (5 uM), and DZ-003 (5 uM) over 12 hours, and the oxygen consumption rate (OCR) from respiration and extracellular acidification rate (ECAR) from glycolysis of the live cells is determined in a metabolic chamber in a 24-well plate format using a Seahorse XF analyzer (Seahorse XFe24, Agilent, CA). Reagents (drugs/controls) are added to the wells just before start of the recording at time 0, and the effect of the drugs on the basal mitochondrial function is recorded by the analyzer for about 12 hours (720 minutes). All reagents used are Agilent reagents. Cells are seeded and cultured in Seahorse XF RPMI Medium, pH 7.4. The seeding rate is 8x10⁵ for the cells to reach about 90% confluency within about 24 hours when the analysis is started. The protocol is essentially performed as per the standard protocol of the manufacturer, removing the medium, washing the cells once with XF Real-Time ATP Rate Assay Medium, then starting the measurement as per the instrument's pre-programming and subsequent real-time measurements of OCAR/ECAR. The results for DZ3a are shown in **FIG. 5C** **(OCR).** Preliminary experiments indicate that the THAD as described herein may have an increased effect compared to DZ3a.
**Example 5D:** JC-1 staining and measuring JC-1 fluorescence is performed by flow cytometry to detect the ratio of green and red fluorescence. C4-2 MDVR cells are treated with vehicle (negative control), DHA (5 uM), and DZ-003 (5 uM) for 16h, followed by JC-1 staining (2uM) at 37 °C for 30 min, to determine the mitochondrial membrane potential by flow cytometry where JC-1 remains as a monomer in the cytosol that emits green fluorescence as an indication of depolarized membrane potential, and JC-1 forms aggregates in intact mitochondria that emits red fluorescence. DZ3a induces depolarization of mitochondrial membrane potential as shown in **FIG. 5D****,** leading to mitochondrial damage and stress to the cells, subsequently followed by cell death. Preliminary experiments indicate that the THAD as described herein may provide a similar or increased effect.
**Example 5E:** Cancer cells (here C4-2B MDVR) are pretreated with OATP inhibitor telmisartan (TMS), mevalonate (MVA), Midivi (Drp-1 inhibitor), and necrostatin-1 (Nec-1) for 2h before treatment with vehicle, DHA, DZ-003 and/or a THAD (e.g. 5 uM each) for 6h. Cells are subjected to annexin v staining (10 ul/10⁶ cells) in the dark at RT for 15-20 min followed by PI staining at 1 mg/ml to determine the apoptosis of the cells based on the PI (red) and Annexin V (green) fluorescence by flow cytometry analysis using SONY SA3800 Spectral Analyzer. Results of DZ-003 compared to DHA are shown in **FIG. 5E****.** DZ-003 induced cell death (upper-left quadrant; green population indicates dead cells) more prominently compared to apoptosis (upper-right quadrant; pink population indicates late apoptosis). The vehicle and DHA treated cells remain viable (lower-left quadrant; blue population indicates live cells). DZ-003-induced cell death can be blocked by mitochondrial fission (Midivi) and necroptosis inhibitor (Nec-1), as shown by a shift in the cell population, i.e. from green to more of the blue cell population, with some of the purple early apoptotic cell population (lower-right quadrant). DZ-003-induced cell death can be partially inhibited by an OATP inhibitor (TMS) but not so much by mevalonic acid (MVA). These results indicate mechanisms that contribute to DZ-003-induced cancer cell death.
**Example 5F:** Cancer cells (here MDVR) are treated with DZ1, DHA and DZ-003 for 8h, followed by C11-BODIPY staining for 30 min at 37°C (lipid peroxidation) or MitoSox staining for 10 min at 37°C (mitochondrial ROS) and analyzed with flow cytometry. Flow cytometry analysis is performed as described in **example 5E** above. DZ3a induces lipid peroxidation (as determined by decreased C11-BODIPY fluorescence, red) and mitochondrial ROS (as determined by increased Mitosox fluorescence, red) as shown in **FIG. 5F****.** Preliminary experiments indicate that the THAD as described herein may provide a similar or increased effects.
**Example 5G:** Cancer cells (here MDVR) are treated with DZ1, DHA and/or a THAD (e.g. 6uM each) for 8h, followed by monobromobimane (mBBr) staining at 40 uM for 30 min at 37 °C, and analyzed with flow cytometry to determine the cellular GSH levels. Flow cytometry analysis of mBBr fluorescence (green) is performed as described in **example 5E** above. Decreased cellular GSH levels as indicated by a decreased mBBr fluorescence for DZ-003 are shown in **FIG. 5G****.** Preliminary experiments indicate that the THAD as described herein may provide a similar or increased effect.
**Example 6A, Synthesis of DZ1-DHA-ether (DZ3c, compound 5):** DZ1-hydroxyethylamino **4** (500 mg, 0.67 mmol) and dihydroartemisinin/DHA **2** (228 mg, 0.81 mmol) are dissolved in methylene chloride ("CH₂Cl₂", 10 ml) with stirring at 0°C. Boron trifluoride diethyl etherate ("BF₃.Et₂O", 0.1 ml) is added and the mixture is stirred about 18 hours at room temperature (RT) to afford a dark green solution. Ethyl ether (40 ml) is added to the reaction mixture. The precipitate is collected and dried under vacuum. The crude product is dissolved in 3 ml of methanol and purified by C18-RP silica column chromatography elution with methanol-water. The major green band is collected and the solvents are removed under reduced pressure. DZ1-DHA ether **5** is obtained as a dark green solid 231 mg (34%). Mass spectrum (ESI) m/z 1014.50 [M+H]⁺. An illustrative reaction scheme is shown in **FIG. 6A****.**
**Example 6B, Synthesis of MHI148-bis-DHA ester (DZ3b, compound 7):** To a solution of MHI-148 **6** (200 mg, 0.28 mmol) in methylene chloride (8 ml) are added: dihydroartemisinin/DHA **2** (191 mg, 0.67 mmol), 1-ethyl-3-(3-dimethyllaminopropyl) carbodiie hydrochloride (EDC) (161 mg, 0.84 mmol) and 4-dimethylaminopyridine (DMAP) (20 mg, 0.16 mmol). The mixture is stirred about 18 hours at RT to afford a dark green solution. Ethyl ether (40 ml) is added to the reaction mixture. The precipitate is collected and dried under vacuum. The crude product is dissolved in 3 ml of methylene chloride ("CH₂Cl₂") and purified by silica gel column chromatography elution with CH₂Cl₂ and methanol/CH₃OH (50:1). The major green band is collected and the solvents are removed under reduced pressure. MHI148-bis-DHA ester **7** is obtained as a dark green solid 129 mg (38%). Mass spectrum (ESI) m/z 1215.67 [M+H]⁺. An illustrative reaction scheme is shown in **FIG. 6B****.**
**Example 6C, Synthesis of DZ1a-bis-DHA ether (DZ3d, compound 9):** DZ1a **8** (500 mg, 0.68 mmol) and dihydroartemisinin **2** (463 mg, 1.63 mmol) are dissolved in methylene chloride (20 ml) with stirring at 0 °C. Boron trifluoride etherate (BF₃.Et₂O, 0.2 ml) is added and the mixture is stirred for 18 hours at RT to afford a dark green solution. Ethyl ether (80 ml) is added to the reaction mixture. The resulting precipitate is collected and dried under vacuum. The resulting crude product is dissolved in 3 ml of methylene chloride and purified by flash silica column chromatography elution with methylene chloride-methanol. The major green band is collected, and the solvents are removed under reduced pressure. DZ1a-bis-DHA-ether **9** is obtained as a dark green solid. An illustrative reaction scheme is shown in **FIG. 6C****.**
**Example 6D, Synthesis of DZ1b-DHA carbamate conjugate (DZ3e, compound 13):** To dihydroartemisinin **2** (100 mg, 0.35 mmol) in dry CH₂Cl₂ (10 ml) at RT is added 1, 1'-carbonyldiimidazole **10** (68 mg, 0.42 mmol). The resulting mixture is stirred for 10 min, then DZlb **12** (271 g, 0.35 mmol) is added. The reaction is allowed to stir at RT for 15 h, then ethyl ether (50 ml) is added and the reaction mixture is filtered. The resulting crude product is dissolved in 3 ml of methanol and purified by C18-RP silica column chromatography elution with methanol-water. The major green band is collected, and the solvents are removed under reduced pressure to afford DZ-DHA carbamate conjugate **13** as a dark green solid. An illustrative reaction scheme is shown in **FIG. 6D****.**
**Example 6E, Synthesis of DZ1c-bis-DHA-carbamate conjugate (DZ3f, compound 15):** To dihydroartemisinin **2** (200 mg, 0.70 mmol) in dry CH₂Cl₂ (20 ml) at RT is added 1,1'-carbonyldiimidazole **10** (136 mg, 0.84 mmol). The mixture is stirred for 10 min, then DZlc **14** (234 g, 0.32 mmol) is added. The reaction is allowed to stir at RT for 15 h, then ethyl ether (50 ml) is added and the reaction mixture is filtered. The resulting crude product is dissolved in 3ml of methanol and purified by C18-RP silica column chromatography elution with methanol-water. The major green band is collected, and the solvents are removed under reduced pressure to afford DZ1c-bis-DHA-carbamate conjugate **15** as a dark green solid. An illustrative reaction scheme is shown in **FIG. 6E****.**
**Example 6F, Synthesis of DZ1b-DHA thiocarbamate conjugate (DZ3g, compound 17):** Thiophosgene (27 µl, 0.35 mmol) is added with stirring to a solution of DZlb **12** (47.8 mg, 0.062 mmol) in dry Tetrahydrofuran (THF, 5 ml). The reaction mixture is stirred at RT for 1.5 h. Methylene chloride 20 ml is added and the mixture is washed with saturated NaHCO₃ (1ml×3) followed by water (1ml×3). The organic layer is dried over MgSO₄ and the solvent is evaporated under reduced pressure to give HMCD-isothiocyanate **16** as a dark green solid. Compound **16** and dihydroartemisinin **2** (1.2 eq) are dissolved in 10 ml of dry THF, then triethyl amine (2 eq) is added. The resulting reaction mixture is heated for 3 h at 65°C. Then ethyl ether is added (50 ml) and the reaction mixture is filtered. The resulting crude product is dissolved in 3ml of methanol and purified by C18-RP silica column chromatography elution with methanol-water. The major green band is collected, and the solvents are removed under reduced pressure to afford DZ1b-DHA thiocarbamate conjugate 17 as a dark green solid.
**Example 6G, Synthesis of DZ1c-bis-DHA thiocarbamate conjugate (DZ3h, compound 19):** Thiophosgene (54 µL, 0.70 mmol) is added with stirring to a solution of DZlc **14** (47.8 mg, 0.062 mmol) in dry THF (5 ml). The reaction mixture is stirred at RT for 1.5 h, then methylene chloride (20 ml) is added and the resulting mixture is washed three times with saturated NaHCO₃ (1ml×3) followed by water (1ml×3). The organic layer is dried over MgSO₄ and the solvent is evaporated under reduced pressure to give MHI148-bis-isothiocyanate **18** as a dark green solid. Compound **18** and dihydroartemisinin **2** (2.4 eq) are dissolved in 15 ml of dry THF, and triethyl amine (4 eq) is added. The resulting reaction mixture is heated for 3 h at 65°C, then ethyl ether is added (75 ml) and the reaction mixture is filtered. The crude product dissolved in 3 ml of methanol and purified by C18-RP silica column chromatography elution with methanol-water. The major green band is collected, and the solvents are removed under reduced pressure to afford DZ1c-bis-DHA carbamate conjugate **19** as a dark green solid.
**Example 6H,** Comparative example (DZ1-artemisinin ester, "DZ3a"): DZ1-artemisinin ester (referred to herein as "DZ-DHA" or "DZ0003") may be synthesized as follows. To a solution of DZ1 (250 mg, 0.35 mmol) in methylene chloride (10 ml) are added dihydroartemisinin (DHA, 110 mg, 0.39 mmol), 1-ethyl-3-(3-dimethyllaminopropyl) carbodiie hydrochloride (EDC) (82 mg, 0.43 mmol) and 4-dimethylaminopyridine (DMAP) (20 mg, 0.16 mmol). The mixture is stirred 15 hours at RT to afford a dark green solution. Ethyl ether (40 ml) is added to the reaction mixture. The precipitate is collected and dried under vacuum. The crude product is dissolved in 3 ml of methanol and purified by C18-RP silica column chromatography elution with methanol-water (from 20% to 80% methanol). The major green band is collected and the solvents are removed under reduced pressure. The DZ1-DHA ester conjugate is obtained as a dark green solid 179 mg (52%). Mass spectrum (ESI) m/z 971.46 [M+H]+.

It should be noted that the features illustrated in the drawings and examples are not necessarily drawn to scale, and features of one embodiment may be employed with other embodiments as the skilled artisan would recognize, even if not explicitly stated herein. Descriptions of well-known components and techniques may be omitted so as to not unnecessarily obscure the embodiments.

While multiple embodiments are disclosed, still other embodiments of the present invention will become apparent to those skilled in the art from this detailed description. The invention is capable of myriad modifications in various obvious aspects, all without departing from the spirit and scope of the present invention. Accordingly, the drawings and descriptions are to be regarded as illustrative in nature rather than restrictive.

Further aspects and/or embodiments of the invention are described in the following clauses:
Clause 1: A tumor-homing dihydroartemisinin derivative (THAD) shown in any of the formulae selected from formulae FI, FII, FIII and FIV below: 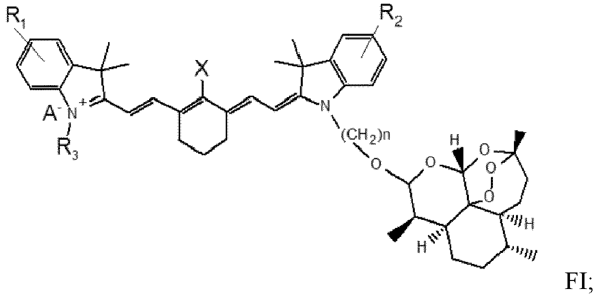 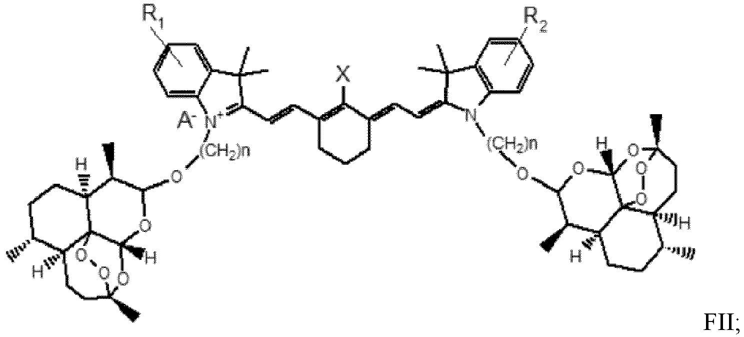 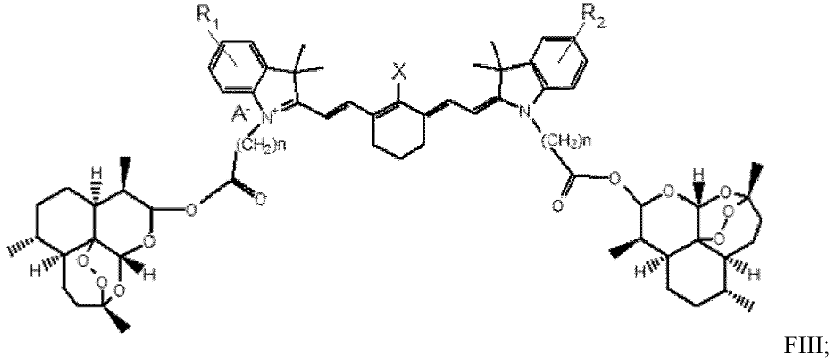 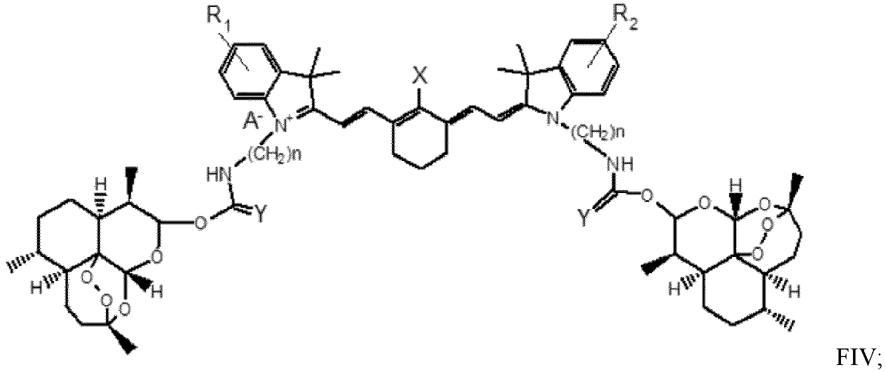
   wherein X is a halogen residue;
   wherein n is independently selected from the group consisting of 2, 3, 4, 5, 6, 7, 8, 9, 10, 11, 12, 13, 14, 15, 16, 17 ,18, 19, 20;
   wherein the A⁻ group is a pharmaceutically acceptable negatively charged anion;
   wherein R₁ and R₂ are residues independently selected from the group consisting of: hydrogen, C₁-C₂₀ alkyl, sulphonate, C₁-C₂₀ alkylcarboxyl, C₁-C₂₀ alkylamino, C₁-C₂₀ aryl, - SO₃H, -PO₃H, -OH, -NH₂, and a halogen residue;
   wherein R₃ of formula FI is a residue selected from the group consisting of: C₁-C₂₅ alkyl, C₅-C₂₅ aryl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ co-alkynyl, a PEGyl polyethylene chain with (-CH₂-CH₂-O-)₂₋₂₀, a PEGylcarboxylate with (-CH₂-CH₂-O-)₂₋₂₀, a co-PEGylaminium with (-CH₂-CH₂-O-)₂₋₂₀, a ω-acyl-NH, a ω- acyl-lysinyl-, a ω -acyl-triazole, a ω- PEGylcarboxyl-NH- with (-CH₂-CH₂-O-)₂₋₂₀, a ω-PEGylcarboxyl-lysinyl with (-CH₂-CH₂-O-)₂₋₂₀, and a ω-PEGylcarboxyl-triazole with (-CH₂-CH₂-O-)₂₋₂₀;
   and wherein Y of formula FIV is independently selected from O and S.
Clause 2: The THAD of clause 1 wherein the THAD is selected from the group consisting of a THAD of formula FI and FII.
Clause 3: The THAD of clause 1 wherein the THAD is selected from the group consisting of a THAD of formula FII, FIII and FIV.
Clause 4: The THAD of clause 1, wherein X is Cl.
Clause 5: The THAD of clause 1, wherein R₁ and R₂ are H.
Clause 6: The THAD of clause 1, wherein R₃ is a -(CH₂)n-SO₃⁻ alkylsulphonate residue, and wherein n of R₁ is selected from 2, 3, 4, 5, 6, 7 and 8.
Clause 7: A pharmaceutical composition comprising one or more THAD and one or more pharmaceutical excipient, wherein the one or more THAD is selected from the group consisting of:
   a) a THAD of formula FI below: 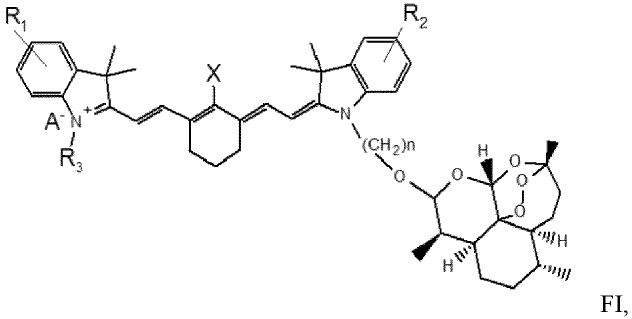
      wherein X is a halogen residue;
      wherein n is independently selected from the group consisting of 2, 3, 4, 5, 6, 7, 8, 9, 10, 11, 12, 13, 14, 15, 16, 17 ,18, 19, 20;
      wherein the A⁻ group is a pharmaceutically acceptable negatively charged anion;
      wherein R₁ and R₂ are residues independently selected from the group consisting of: hydrogen, C₁-C₂₀ alkyl, sulphonate, C₁-C₂₀ alkylcarboxyl, C₁-C₂₀ alkylamino, C₁-C₂₀ aryl, - SO₃H, -PO₃H, -OH, -NH₂, and a halogen residue;
      and wherein R₃ is a residue selected from the group consisting of: C₁-C₂₅ alkyl, C₅-C₂₅ aryl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ co-alkynyl, a PEGyl polyethylene chain with (-CH₂-CH₂-O-)₂₋₂₀, a PEGylcarboxylate with (-CH₂-CH₂-O-)₂₋₂₀, a co-PEGylaminium with (-CH₂-CH₂-O-)₂₋₂₀, a co-acyl-NH, a co- acyl-lysinyl-, a co -acyl-triazole, a co- PEGylcarboxyl-NH- with (-CH₂-CH₂-O-)₂₋₂₀, a ω-PEGylcarboxyl-lysinyl with (-CH₂-CH₂-O-)₂₋₂₀, and a ω- PEGylcarboxyltriazole with (-CH₂-CH₂-O-)₂₋₂₀;
   b) a THAD of formula FII below: 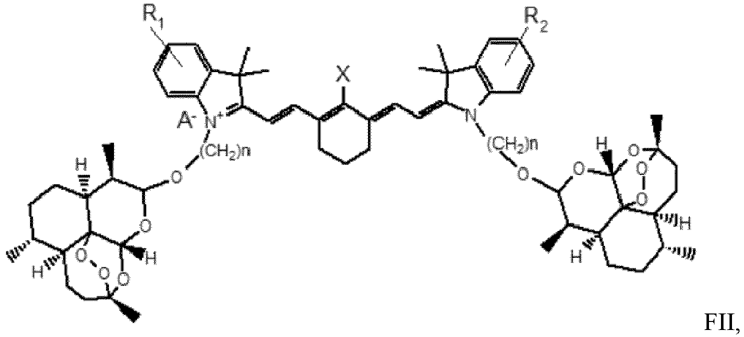 and wherein X, n, A⁻, R₁, and R₂ are defined as for FI in (a) above;
   c) a THAD of formula FIII below: 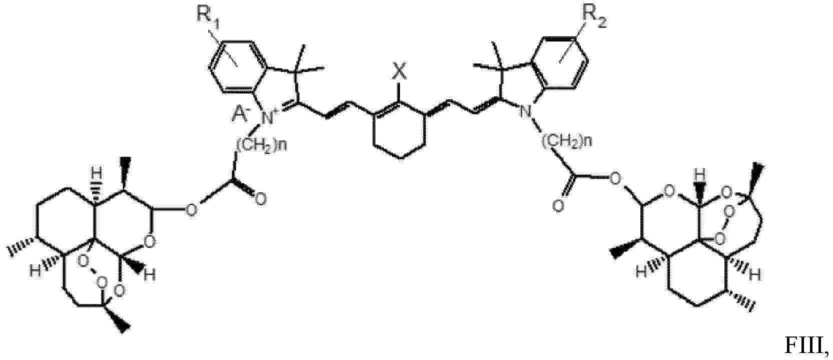 and wherein X, n, A⁻, R₁, and R₂ are defined as for FI in (a) above;
   d) a THAD of formula FIV below: 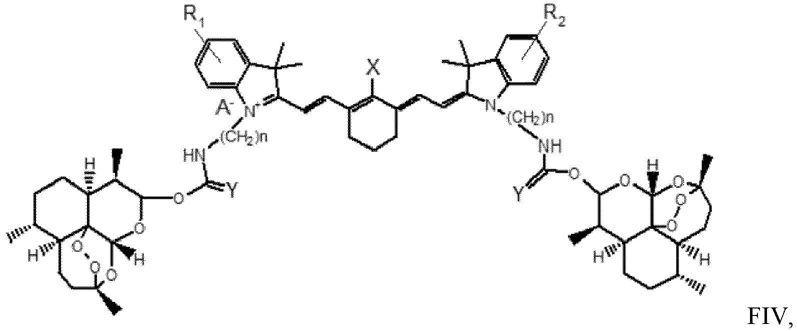
      wherein Y is selected from the group consisting of O and S,
      and wherein X, n, A⁻, R₁, and R₂ are defined as for FI in (a) above;
   e) a THAD as defined in (a), wherein X is Cl;
   f) a THAD as defined in (b), wherein X is Cl;
   g) a THAD as defined in (c), wherein X is Cl;
   h) a THAD as defined in (d), wherein X is Cl;
   i) a THAD as defined in (a), wherein R₁ and R₂ are H.
   j) a THAD as defined in (b), wherein R₁ and R₂ are H.
   k) a THAD as defined in (c), wherein R₁ and R₂ are H.
   l) a THAD as defined in (d), wherein R₁ and R₂ are H.
   m) a THAD as defined in (a), wherein R₃ is a -(CH₂)n-SO₃⁻ alkylsulphonate residue and wherein n of R₃ is selected from 2, 3, 4, 5, 6, 7 and 8;
   n) a THAD as defined in (a), wherein R₃ is a -(CH₂)₄-SO₃⁻ alkylsulphonate residue.
Clause 8: The pharmaceutical composition of clause 7 wherein the THAD is an ether selected from an ether of formulae FI and FII as defined in (a) and (b).
Clause 9: The pharmaceutical composition of clause 7 wherein the THAD is a bis-DHA compound selected from a THAD of formulae FII, FIII and FIV as defined in (b), (c) and (d).
Clause 10: The pharmaceutical composition of clause 7 wherein the composition is provided in a dosage form which is adapted to provide a low dosage of up to 2 mg/kg of the one or more THAD or less upon administration of the dosage form.
Clause 11: A method of treating cancer wherein one or more THAD is administered to a patient in need thereof in amounts sufficient to inhibit cancer cell or pre-cancerous cell growth or induce apoptosis in cancer or pre-cancerous cells in the patient, and wherein the one or more THAD is selected from the group consisting of:
   a) a THAD of formula FI below: 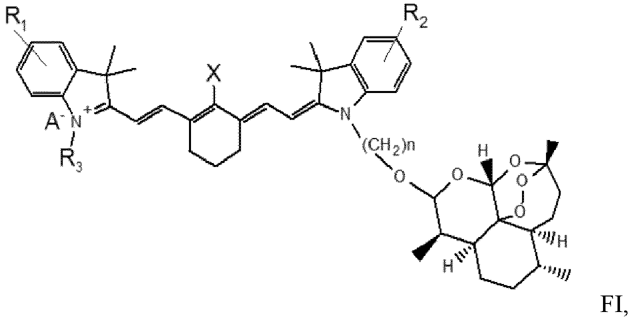
      wherein X is a halogen residue;
      wherein n is independently selected from the group consisting of 2, 3, 4, 5, 6, 7, 8, 9, 10, 11, 12, 13, 14, 15, 16, 17 ,18, 19, 20;
      wherein the A⁻ group is a pharmaceutically acceptable negatively charged anion;
      wherein R₁ and R₂ are residues independently selected from the group consisting of: hydrogen, C₁-C₂₀ alkyl, sulphonate, C₁-C₂₀ alkylcarboxyl, C₁-C₂₀ alkylamino, C₁-C₂₀ aryl, - SO₃H, -PO₃H, -OH, -NH₂, and a halogen residue;
      and wherein R₃ is a residue selected from the group consisting of: C₁-C₂₅ alkyl, C₅-C₂₅ aryl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ co-alkynyl, a PEGyl polyethylene chain with (-CH₂-CH₂-O-)₂₋₂₀, a PEGylcarboxylate with (-CH₂-CH₂-O-)₂₋₂₀, a co-PEGylaminium with (-CH₂-CH₂-O-)₂₋₂₀, a ω-acyl-NH, a ω- acyl-lysinyl-, a ω -acyl-triazole, a ω- PEGylcarboxyl-NH- with (-CH₂-CH₂-O-)₂₋₂₀, a ω-PEGylcarboxyl-lysinyl with (-CH₂-CH₂-O-)₂₋₂₀, and a ω- PEGylcarboxyltriazole with (-CH₂-CH₂-O-)₂₋₂₀;
   b) a THAD of formula FII below: 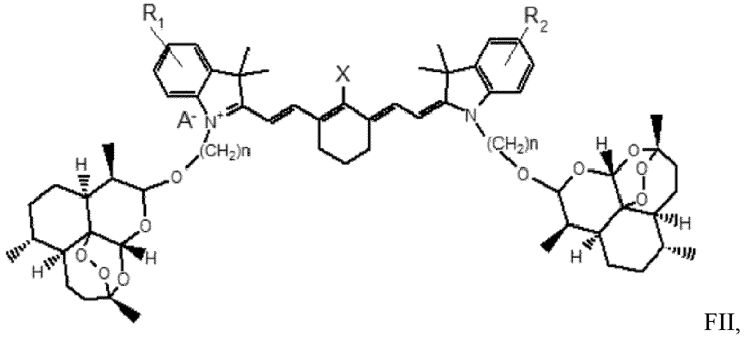 and wherein X, n, A⁻, R₁, and R₂ are defined as for FI in (a) above;
   c) a THAD of formula FIII below: 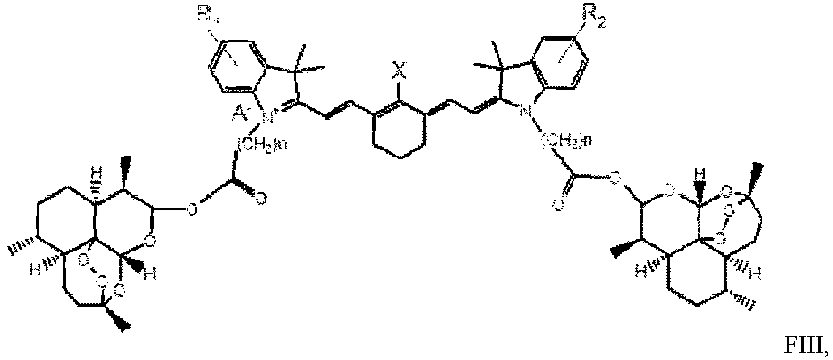 and wherein X, n, A⁻, R₁, and R₂ are defined as for FI in (a) above;
   d) a THAD of formula FIV below: 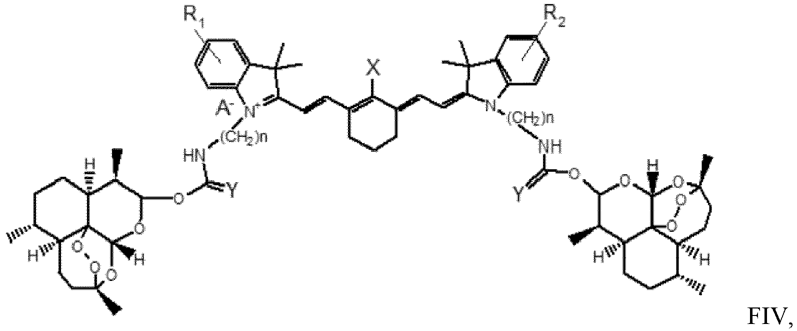
      wherein Y is selected from the group consisting of O and S,
      and wherein X, n, A⁻, R₁, and R₂ are defined as for FI in (a) above;
   e) a THAD as defined in (a), wherein X is Cl;
   f) a THAD as defined in (b), wherein X is Cl;
   g) a THAD as defined in (c), wherein X is Cl;
   h) a THAD as defined in (d), wherein X is Cl;
   i) a THAD as defined in (a), wherein R₁ and R₂ are H.
   j) a THAD as defined in (b), wherein R₁ and R₂ are H.
   k) a THAD as defined in (c), wherein R₁ and R₂ are H.
   l) a THAD as defined in (d), wherein R₁ and R₂ are H.
   m) a THAD as defined in (a), wherein R₃ is a -(CH₂)n-SO₃⁻ alkylsulphonate residue and wherein n of R₃ is selected from 2, 3, 4, 5, 6, 7 and 8;
   n) a THAD as defined in (a), wherein R₃ is a -(CH₂)₄-SO₃⁻ alkylsulphonate residue.
Clause 12: The method of clause 11, wherein the one or more THAD is administered to the patient in a dosage of up to 2 mg/kg of the one or more THAD or less.
Clause 13: The method of clause 11, wherein the one or more THAD is co-administered in a coordinated administration schedule together with one or more secondary drug, and wherein the one or more secondary drug is selected from the group consisting of: a hormonal antagonist, an anti-androgenic drug, Abiraterone acetate, Enzalutamide, a chemotherapeutic drug, Docetaxel, Paclitaxel, and Cabazitaxel.
Clause 14: The method of clause 11, wherein the one or more THAD is administered to a patient whose cancer cells, pre-cancerous lesions, tissues, tumors or metastases are identified to carry one or more genetic aberration in one or more gene encoding for one or more tyrosine kinase receptor, selected from the group comprising: epidermal growth factor receptor tyrosine kinase (EGFR), Anaplastic lymphoma kinase receptor (ALF), and Proto-oncogene tyrosine-protein kinase (ROS or ROS1).
Clause 15: The method of clause 10, wherein the one or more THAD is administered to a patient whose cancer cells, pre-cancerous lesions, tumors or metastases have acquired resistance to one or more tyrosine kinase inhibitor (TKI), including a patient who received prior TKI treatment with one or more TKI prior to THAD administration and whose response to the prior TKI treatment is therapeutically insufficient.
Clause 16: The method of clause 15, wherein the TKI is selected from the group consisting of an epidermal growth factor receptor tyrosine kinase inhibitor (EGFR-TKI), an ALK tyrosine kinase receptor inhibitor (ALK-TKI), an inhibitor to Proto-oncogene tyrosine-protein kinase ROS (ROS-TKI), Gefitinib, Icotinib, Erlotinib, Brigatinib, Dacomitinib, Lapatinib, Vandetanib, Afatinib, Osimertinib (AZD9291), CO-1686, HM61713, Nazartinib (EGF816), Olmutinib, PF-06747775, YH5448, Avitinib (AC0010), Rociletinib, and Cetuximab.
Clause 17: The method of clause 10, wherein the patient is suffering from a drug-resistant cancer as determined by drug exposure or genetic testing, the drug-resistant cancer selected from the group comprising: kidney cancer, prostate cancer, pancreatic cancer, lung cancer, non-small cell lung carcinoma (NSCLC; NSCLC may include squamous-cell carcinoma, adenocarcinoma (mucinous cystadenocarcinoma), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma), SCLC (small cell lung carcinoma), combined small-cell carcinoma, non-carcinoma cancers of the lung (sarcoma, lymphoma, immature teratoma, and melanoma), kidney cancer, lymphoma, colorectal cancer, skin cancer, HCC cancer, and breast cancer, squamous-cell carcinoma of the lung, anal cancers, glioblastoma, epithelial tumors of the head and neck, and other cancers.
Clause 18: The method of clause 10, wherein the patient is a patient suffering from a drug-resistant lung cancer as determined by drug exposure or genetic testing, the drug resistant lung cancer selected from the group comprising: small cell carcinoma lung cancer (SCCLC), non-small cell lung carcinoma (NSCLC), combined small-cell carcinoma, squamous-cell carcinoma, adenocarcinoma (AC, mucinous cystadenocarcinoma, MCACL), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma, non-carcinoma cancer of the lung, sarcoma, lymphoma, immature teratoma, and melanoma.
Clause 19: The method of clause 10, wherein the one or more THAD and one or more HMCD-DHA-mono ester, are co-administered in a coordinated administration schedule of one or more combined dosage forms, wherein each dosage form comprises:
   a) one or more HMCD-DHA-mono-ether and one or more HMCD-DHA-mono ester;
   b) one or more HMCD-DHA-bis-ether and one or more HMCD-DHA-mono-ester;
   c) one or more HMCD-DHA-bis-carbamate and one or more HMCD-DHA-mono-ester;
   d) one or more HMCD-DHA-bis-thiocarbamate and one or more HMCD-DHA-mono-ester;
   and wherein the dosage form further comprises one or more pharmaceutical excipient.
Clause 20: The method of clause 19, wherein the schedule includes administration of a loading dose administered at least one or more hour prior to administration of one or more maintenance dose;
   wherein the loading dose consists of a separate dosage form that comprises one or more loading compound and one or more pharmaceutical excipient, and does not comprise the one or more maintenance compound;
   and wherein the one or more maintenance dose consists of a dosage form that comprises the one or more maintenance compound and one or more pharmaceutical excipient, and optionally comprises the loading compound;
   and wherein loading and maintenance compounds are thus administered sequentially in time, and selected from the following:
   a) a loading dose of one or more HMCD-DHA-mono-ether and a maintenance dose of one or more HMCD-DHA-mono ester;
   b) a loading dose of one or more HMCD-DHA-bis-ether and a maintenance dose of one or more HMCD-DHA-mono-ester;
   c) a loading dose of one or more HMCD-DHA-bis-carbamate and a maintenance dose of one or more HMCD-DHA-mono-ester;
   d) a loading dose of one or more HMCD-DHA-bis-thiocarbamate and a maintenance dose of one or more HMCD-DHA-mono-ester.
Clause 21: A HMCD dye selected from the group of dyes shown below: 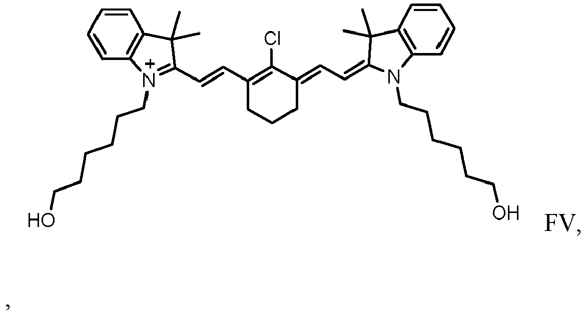 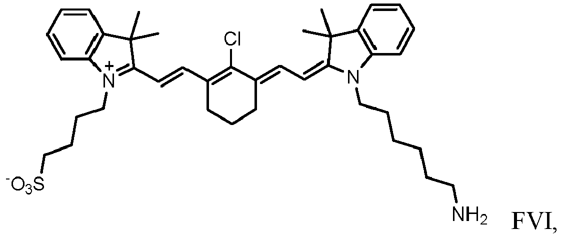 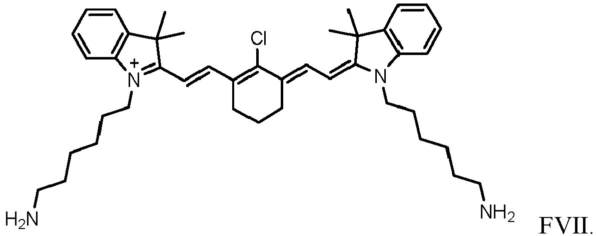
Clause 22: A process of making a HMCD-drug conjugate wherein a HMCD is reacted with one or more further educts to form the conjugate, wherein the one or more further educts comprise a drug, or a derivative of the drug, and wherein the HMCD is selected from: 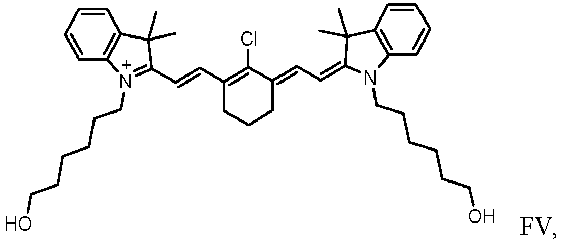 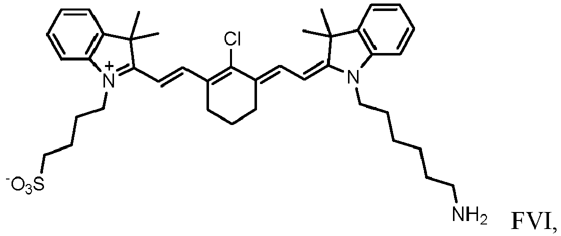 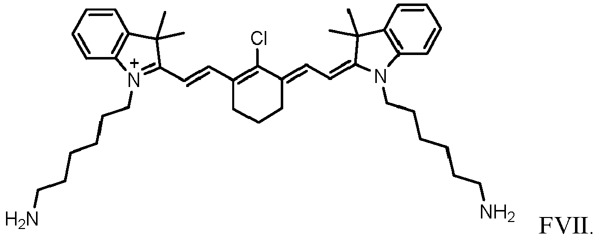
Clause 23: The process of clause 22 wherein the conjugate is a THAD, wherein a HMCD is reacted with one or more further educts to form the THAD, wherein the one or more further educts comprise artemisinin or dihydroartemisinin (DHA), or a derivative of artemisinin or dihydroartemisinin (DHA), and wherein the HMCD is selected from: 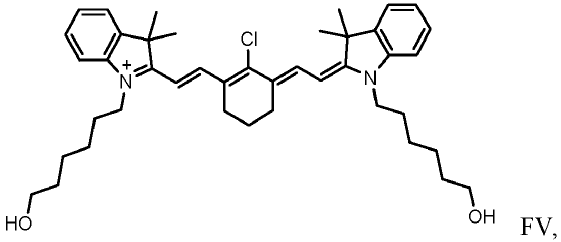 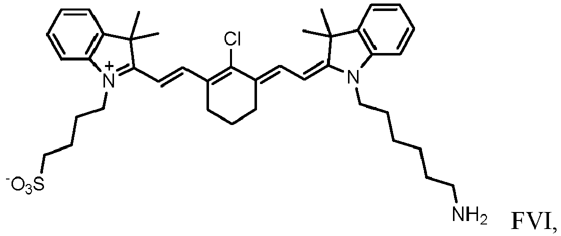 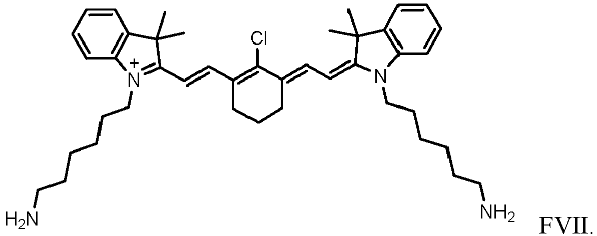
Clause 24: A DRG-HMCD drug-dye conjugate wherein the HMCD residue is selected from the group consisting of DZ1a, DZ1b and DZ1c, wherein the drug and the HMCD are liked by ether, ester, carbamate or thiocarbamate linkage, wherein the linkage may be a mono-linkage to one drug molecule, or a bis-linkage to two drug molecules, and wherein the DRG-HMCD is selected from the group comprising the following conjugate types:
   a) DZ1a-DRG-ether, DZ1a-bis-DRG-ether, DZ1a-DRG-ester, DZ1a-bis-DRG-ester, DZ1a-DRG-carbamate, DZ1a-bis-DRG-carbamate, DZ1a-DRG-thiocarbamate, DZ1a-bis-DRG-thiocarbamate;
   b) DZ1b-DRG-ether, DZ1b-bis-DRG-ether, DZ1b-DRG-ester, DZ1b-bis-DRG-ester, DZ1b-DRG-carbamate, DZ1b-bis-DRG-carbamate, DZ1b-DRG-thiocarbamate, DZ1b-bis-DRG-thiocarbamate; and
   c) DZ1c-DRG-ether, DZ1c-bis-DRG-ether, DZ1c-DRG-ester, DZ1c-bis-DRG-ester, DZ1c-DRG-carbamate, DZ1c-bis-DRG-carbamate, DZ1c-DRG-thiocarbamate, DZ1c-bis-DRG-thiocarbamate.
Clause 25: A THAD wherein the THAD conjugate of a dye residue conjugated to a DHA residue, wherein the HMCD dye residue is selected from the group consisting of DZla, DZlb and DZ1c, and wherein the THAD is selected from the group of the following conjugate types:
   a) DZ1a-DHA-ether, DZ1a-bis-DHA-ether, DZ1a-DHA-ester, DZ1a-bis-DHA-ester, DZ1a-DHA-carbamate, DZ1a-bis-DHA-carbamate, DZ1a-DHA-thiocarbamate DZ1a-bis-DHA-thiocarbamate;
   b) DZ1b-DHA-ether, DZ1b-bis-DHA-ether, DZ1b-DHA-ester, DZ1b-bis-DHA-ester, DZ1b-DHA-carbamate, DZ1b-bis-DHA-carbamate, DZ1b-DHA-thiocarbamate, DZ1b-bis-DHA-thiocarbamate; and
   c) DZ1c-DHA-ether, DZ1c-bis-DHA-ether, DZ1c-DHA-ester, DZ1c-bis-DHA-ester, DZ1c-DHA-carbamate, DZ1c-bis-DHA-carbamate, DZ1c-DHA-thiocarbamate, DZ1c-bis-DHA-thiocarbamate.
Clause 26: A THAD selected from the group consisting of THAD shown below:

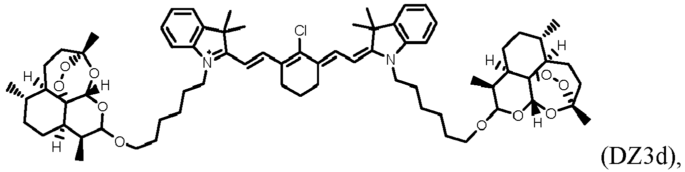

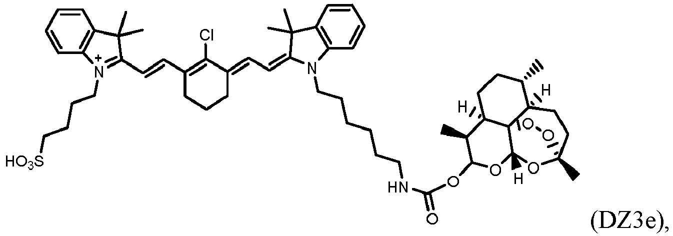

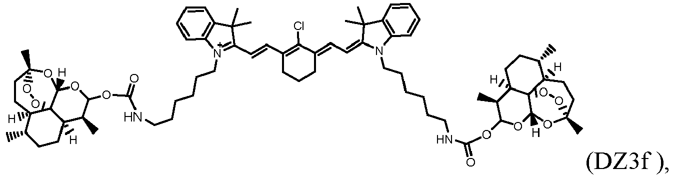

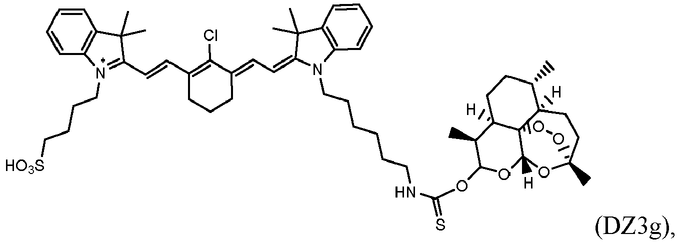

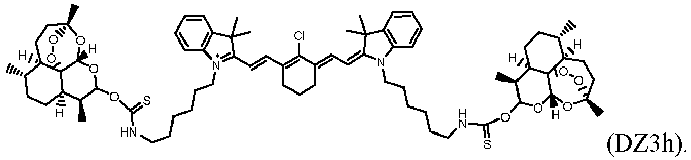

Each of the embodiments defined by the above clauses may be incorporated, where appropriate in the context, into a tumor-homing dihydroartemisinin derivative (THAD) or a process according to the aspects of the invention set out above.

## Claims

1. A tumor-homing dihydroartemisinin derivative (THAD) shown in any of the formulae selected from formulae FI, FII, FIII and FIV below: 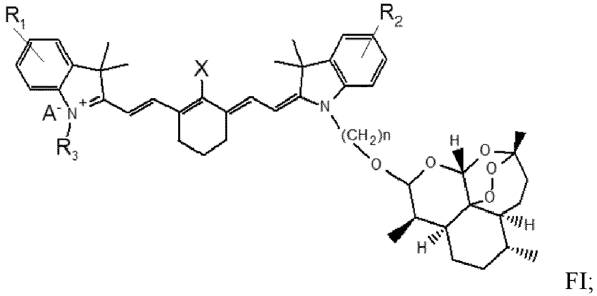 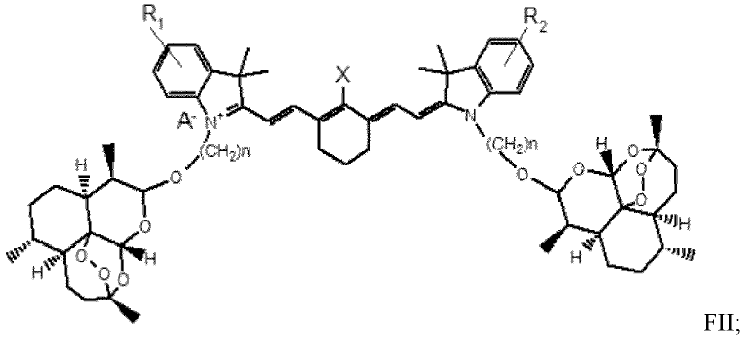 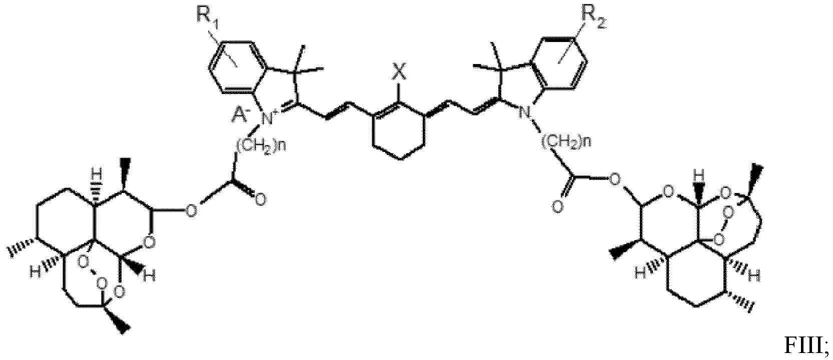 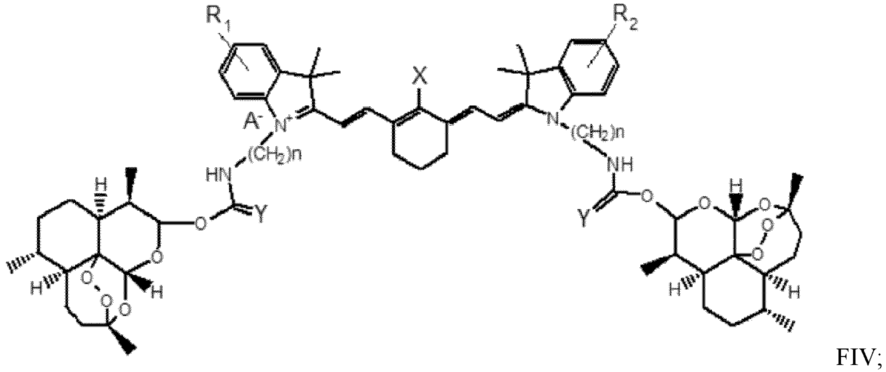
wherein X is a halogen residue;
wherein n is independently selected from the group consisting of 2, 3, 4, 5, 6, 7, 8, 9, 10, 11, 12, 13, 14, 15, 16, 17 ,18, 19, 20;
wherein the A⁻ group is a pharmaceutically acceptable negatively charged anion;
wherein R₁ and R₂ are residues independently selected from the group consisting of: hydrogen, C₁-C₂₀ alkyl, sulphonate, C₁-C₂₀ alkylcarboxyl, C₁-C₂₀ alkylamino, C₁-C₂₀ aryl, -SO₃H, -PO₃H, -OH, -NH₂, and a halogen residue;
wherein R₃ of formula FI is a residue selected from the group consisting of: C₁-C₂₅ alkyl, C₅-C₂₅ aryl, C₁-C₂₅ aralkyl, C₁-C₂₅ alkylsulphonate, C₁-C₂₅ alkylcarboxyl, C₁-C₂₅ alkylamino, C₁-C₂₅ ω-alkylaminium, C₁-C₂₅ ω-alkynyl, a PEGyl polyethylene chain with (-CH₂-CH₂-O-)₂₋₂₀, a PEGylcarboxylate with (-CH₂-CH₂-O-)₂₋₂₀, a ω-PEGylaminium with (-CH₂-CH₂-O-)₂₋₂₀, a ω-acyl-NH, a ω- acyl-lysinyl-, a ω -acyl-triazole, a ω- PEGylcarboxyl-NH- with (-CH₂-CH₂-O-)₂₋₂₀, a ω-PEGylcarboxyl-lysinyl with (-CH₂-CH₂-O-)₂₋₂₀, and a ω-PEGylcarboxyl-triazole with (-CH₂-CH₂-O-)₂₋₂₀; and
wherein Y of formula FIV is independently selected from O and S.

2. A tumor-homing dihydroartemisinin derivative (THAD) as claimed in claim 1 wherein the THAD is selected from the group consisting of:
(i) an ether of formula FI and FII;
(ii) a bis-DHA THAD of formula FII, FIII and FIV;
(iii) a THAD of formula FI, FII, FIII or FIV wherein X is Cl;
(iv) a THAD of formula FI, FII, FIII or FIV wherein R₁ and R₂ are H;
(v) a THAD of formula FI, wherein R₃ is a -(CH₂)n-SO₃⁻ alkylsulphonate residue, and wherein n of R₁ is selected from the group consisting of 2, 3, 4, 5, 6, 7 and 8; and
(vi) a THAD of formula FI, wherein R₃ is a -(CH₂)₄-SO₃⁻ alkylsulphonate residue.

3. A tumor-homing dihydroartemisinin derivative (THAD) as claimed in claim 2, wherein the THAD is (i) an ether of formula FI and FII.

4. A tumor-homing dihydroartemisinin derivative (THAD) as claimed in claim 2, wherein the THAD is (ii) a bis-DHA THAD of formula FII, FIII and FIV.

5. A tumor-homing dihydroartemisinin derivative (THAD) as claimed in claim 2, wherein the THAD is (iii) a THAD of formula FI, FII, FIII or FIV wherein X is Cl.

6. A tumor-homing dihydroartemisinin derivative (THAD) as claimed in any one of claims 1 to 5, wherein the one or more THAD and one or more pharmaceutical excipient are comprised in a pharmaceutical composition.

7. A tumor-homing dihydroartemisinin derivative (THAD) as claimed in any one of claims 1 to 6, wherein the THAD is provided in a dosage form which is adapted to provide a low dosage of up to 2 mg/kg or less of the one or more THAD upon administration of the dosage form.

8. A tumor-homing dihydroartemisinin derivative (THAD) as claimed in any one of claims 1 to 7 for use in treating cancer, wherein optionally one or more THAD is prepared to be administered to a patient in need thereof in amounts sufficient to inhibit cancer cell or pre-cancerous cell growth or induce apoptosis in cancer or pre-cancerous cells in the patient.

9. A tumor-homing dihydroartemisinin derivative (THAD) as claimed in claim 8 wherein the THAD is prepared to be co-administered in a coordinated administration schedule together with one or more secondary drug, and wherein the one or more secondary drug is selected from the group consisting of: a hormonal antagonist, an anti-androgenic drug, Abiraterone acetate, Enzalutamide, a chemotherapeutic drug, Docetaxel, Paclitaxel, and Cabazitaxel.

10. A tumor-homing dihydroartemisinin derivative (THAD) as claimed in any one of claims 1 to 9, wherein the THAD is prepared to be administered to a patient selected from the group comprising:
(i) a patient whose cancer cells, pre-cancerous lesions, tissues, tumors or metastases are identified to carry one or more genetic aberration in one or more gene encoding for one or more tyrosine kinase receptor, selected from the group comprising: epidermal growth factor receptor tyrosine kinase (EGFR), Anaplastic lymphoma kinase receptor (ALF), and Proto-oncogene tyrosine-protein kinase (ROS or ROS1);
(ii) a patient whose cancer cells, pre-cancerous lesions, tumors or metastases have acquired resistance to one or more tyrosine kinase inhibitor (TKI);
(iii) a patient who received prior TKI treatment with one or more TKI prior to THAD administration and whose response to the prior TKI treatment is therapeutically insufficient;
(iv) the patient of (ii) or (iii), wherein the TKI is selected from the group consisting of an epidermal growth factor receptor tyrosine kinase inhibitor (EGFR-TKI), an ALK tyrosine kinase receptor inhibitor (ALK-TKI), an inhibitor to Proto-oncogene tyrosine-protein kinase ROS (ROS-TKI), Gefitinib, Icotinib, Erlotinib, Brigatinib, Dacomitinib, Lapatinib, Vandetanib, Afatinib, Osimertinib (AZD9291), CO-1686, HM61713, Nazartinib (EGF816), Olmutinib, PF-06747775, YH5448, Avitinib (AC0010), Rociletinib, and Cetuximab;
(v) a patient wherein the patient is suffering from a drug-resistant cancer as determined by drug exposure or genetic testing, the drug-resistant cancer selected from the group comprising: kidney cancer, prostate cancer, pancreatic cancer, lung cancer, non-small cell lung carcinoma (NSCLC; NSCLC may include squamous-cell carcinoma, adenocarcinoma (mucinous cystadenocarcinoma), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma), SCLC (small cell lung carcinoma), combined small-cell carcinoma, non-carcinoma cancers of the lung (sarcoma, lymphoma, immature teratoma, and melanoma), kidney cancer, lymphoma, colorectal cancer, skin cancer, HCC cancer, and breast cancer, squamous-cell carcinoma of the lung, anal cancers, glioblastoma, epithelial tumors of the head and neck, and other cancers; and
(vi) a patient suffering from a drug-resistant lung cancer as determined by drug exposure or genetic testing, the drug resistant lung cancer selected from the group comprising: small cell carcinoma lung cancer (SCCLC), non-small cell lung carcinoma (NSCLC), combined small-cell carcinoma, squamous-cell carcinoma, adenocarcinoma (AC, mucinous cystadenocarcinoma, MCACL), large-cell lung carcinoma, rhabdoid carcinoma, sarcomatoid carcinoma, carcinoid, salivary gland-like carcinoma, adenosquamous carcinoma, papillary adenocarcinoma, giant-cell carcinoma, non-carcinoma cancer of the lung, sarcoma, lymphoma, immature teratoma, and melanoma.

11. A tumor-homing dihydroartemisinin derivative (THAD) as claimed in any one of claims 1 to 10, wherein the one or more THAD and one or more HMCD-DHA-mono ester are prepared to be co-administered to a patient in a coordinated administration schedule of one or more combined dosage forms, wherein each dosage form comprises:
a) one or more HMCD-DHA-mono-ether and one or more HMCD-DHA-mono ester;
b) one or more HMCD-DHA-bis-ether and one or more HMCD-DHA-mono-ester;
c) one or more HMCD-DHA-bis-carbamate and one or more HMCD-DHA-mono-ester;
d) one or more HMCD-DHA-bis-thiocarbamate and one or more HMCD-DHA-mono-ester; and
wherein optionally, the dosage form further comprises one or more pharmaceutical excipient.

12. A tumor-homing dihydroartemisinin derivative (THAD) as claimed in claim 11 for use in a coordinated administration schedule, wherein the schedule includes administration of a loading dose prepared to be administered at least one or more hour prior to administration of one or more maintenance dose;
wherein the loading dose consists of a separate dosage form that comprises one or more loading compound and one or more pharmaceutical excipient, and does not comprise the one or more maintenance compound;
wherein the one or more maintenance dose consists of a dosage form that comprises the one or more maintenance compound and one or more pharmaceutical excipient, and optionally comprises the loading compound; and
wherein the one or more loading compound and the one or more maintenance compound are thus prepared to be administered sequentially in time, and selected from the following:
a) a loading dose of one or more HMCD-DHA-mono-ether and a maintenance dose of one or more HMCD-DHA-mono ester;
b) a loading dose of one or more HMCD-DHA-bis-ether and a maintenance dose of one or more HMCD-DHA-mono-ester;
c) a loading dose of one or more HMCD-DHA-bis-carbamate and a maintenance dose of one or more HMCD-DHA-mono-ester; and
d) a loading dose of one or more HMCD-DHA-bis-thiocarbamate and a maintenance dose of one or more HMCD-DHA-mono-ester.

13. A tumor-homing dihydroartemisinin derivative (THAD) as claimed in any one of claims 1 to 12, wherein the HMCD residue is selected from the group consisting of FV (DZ1a), FVI (DZ1b) and FVII (DZ1c) as shown below: 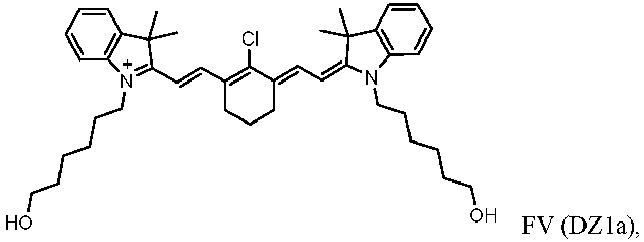 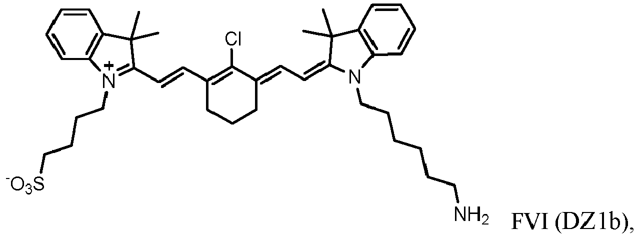 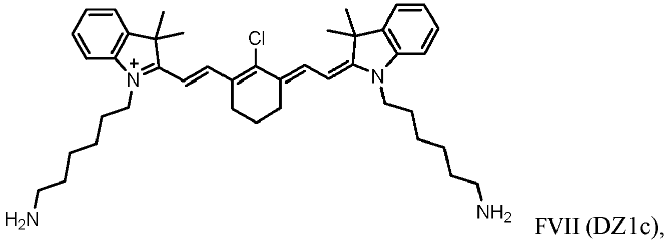 wherein the dye residue is conjugated to a DHA residue, and
wherein the THAD is selected from the group comprising the following conjugate types:
a) DZ1a-DHA-ether, DZ1a-bis-DHA-ether, DZ1a-DHA-ester, DZ1a-bis-DHA-ester, DZ1a-DHA-carbamate, DZ1a-bis-DHA-carbamate, DZ1a-DHA-thiocarbamate DZ1a-bis-DHA-thiocarbamate;
b) DZ1b-DHA-ether, DZ1b-bis-DHA-ether, DZ1b-DHA-ester, DZ1b-bis-DHA-ester, DZ1b-DHA-carbamate, DZ1b-bis-DHA-carbamate, DZ1b-DHA-thiocarbamate, DZ1b-bis-DHA-thiocarbamate; and
c) DZ1c-DHA-ether, DZ1c-bis-DHA-ether, DZ1c-DHA-ester, DZ1c-bis-DHA-ester, DZ1c-DHA-carbamate, DZ1c-bis-DHA-carbamate, DZ1c-DHA-thiocarbamate, DZ1c-bis-DHA-thiocarbamate.

14. A tumor-homing dihydroartemisinin derivative (THAD) as claimed in claim 13, wherein the THAD is selected from the group consisting of a THAD of a formula as shown below:

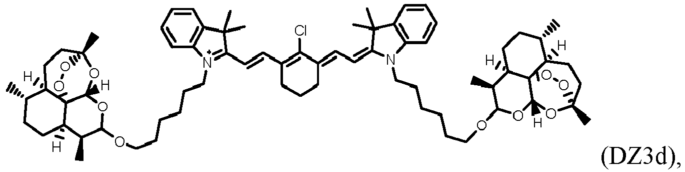

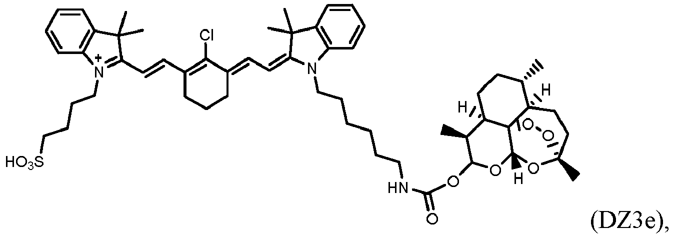

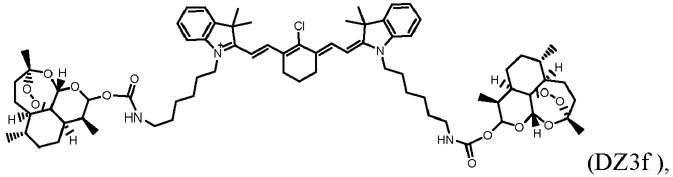

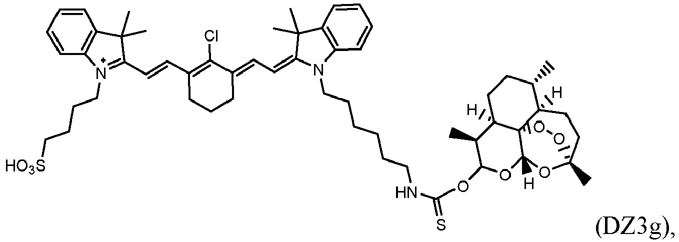

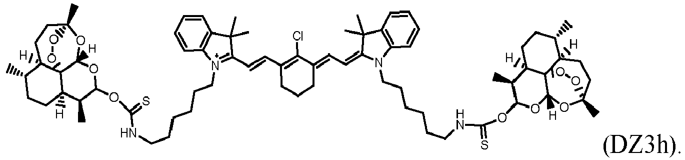

15. A process of making a HMCD-drug conjugate wherein a HMCD dye is reacted with one or more further educts to form the conjugate, wherein the one or more further educts comprise a drug, or a derivative of the drug, and wherein the HMCD dye is selected from: 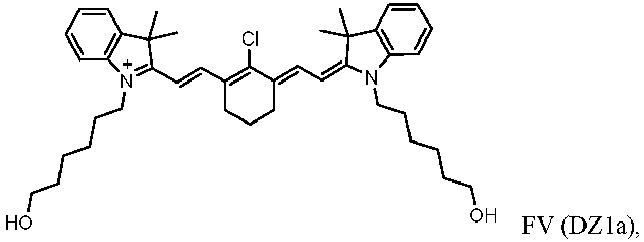 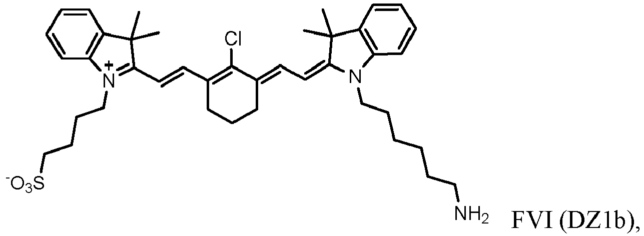 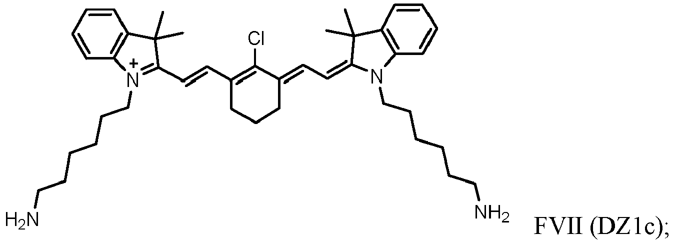 and
wherein the conjugate formed is a THAD,
wherein a HMCD is reacted with one or more further educts to form the THAD, and
wherein the one or more further educts comprise artemisinin or dihydroartemisinin (DHA), or a derivative of artemisinin or dihydroartemisinin (DHA).
